# EUROPÄISCHE PATENTSCHRIFT

(11) **EP 1 044 377 B1**
(45) Veröffentlichungstag und Bekanntmachung des Hinweises auf die Patenterteilung: **18.07.2007**
(21) Anmeldenummer: 98966807.4
(22) Anmeldetag: 24.12.1998
(51) Int. Cl.: G01N 33/569

(54) **VERFAHREN ZUM NACHWEIS VON BORNA-DISEASE-VIRUS (BDV)- INFEKTIONEN**
PROCESS FOR DETECTING BORNA DISEASE VIRUS (BDV) INFECTIONS
PROCEDE POUR LA DETECTION D'INFECTIONS DUES AU VIRUS DE LA MALADIE DE BORNA (BDV)

(30) Priorität: 29.12.1997 DE 19758017
(43) Veröffentlichungstag der Anmeldung: 18.10.2000
(73) Patentinhaber: Ludwig, Hanns, 14163 Berlin (DE)
(72) Erfinder: LUDWIG, Hanns, D-14163 Berlin (DE); BODE, Liv, D-14163 Berlin (DE)
(74) Vertreter: Läufer, Martina
(86) Internationale Anmeldenummer: PCT/DE1998/003793
(87) Internationale Veröffentlichungsnummer: WO 1999/034216

(56) Entgegenhaltungen:
- WO-A-96/21020
- WO-A-98/30238
- US-A- 5 654 401
- BRIESE T ET AL: "Enzyme-linked immunosorbent assay for detecting antibodies to Borna disease virus -specific proteins." JOURNAL OF CLINICAL MICROBIOLOGY, (1995 FEB) 33 (2) 348-51. JOURNAL CODE: HSH. ISSN: 0095-1137., XP000571175 United States
- STITZ L ET AL: "BORNA DISEASE IN RHESUS MONKEYS AS A MODEL FOR UVEO CEREBRAL SYMPTOMS." J MED VIROL, (1980 (RECD 1981)) 6 (4), 333-340. CODEN: JMVIDB. ISSN: 0146-6615., XP002103967

## Beschreibung

Die Erfindung betrifft ein Verfahren zum Nachweis von BDV-Infektionen über den Nachweis in Körperflüssigkeit zirkulierender Immunkomplexe (CICs), ein Verfahren zum Nachweis allgemein von CICs und insbesondere von BDV-spezifischen CICs, sowie ein diagnostischer Kit, der für diese Nachweisverfahren geeignet ist.

BDV-Infektionen kommen bei vielen Nutz- und Haustieren (Pferd, Schaf, Rind, Katze) und dem Menschen vor. BDV ist ein eingehülltes Virus von 90 nm Durchmesser mit einem Genom aus unsegmentierter einzelsträngiger RNA von negativer Polarität, das für 5 Gene kodiert (Größe des Genoms: 8,9 Kilobasen). Zu den verwandten Viren gehören z.B. Tollwutvirus und Masernvirus. Aufgrund genetischer Besonderheiten (Vermehrung im Zellkern der Wirtszelle) ist BDV als der Prototyp einer eigenen Virusfamilie klassifiziert (Bornaviridae). BDV hat eine besondere Präferenz zu den Nervenzellen im limbischen System des Gehirn, einer funktionellen Einheit, in der Verhalten, Emotionen und Gedächtnisleistungen gesteuert werden. Es werden aber auch andere Zelltypen von BDV befallen. Diagnostisch besonders bedeutsam sind die peripheren mononukleären weißen Blutzellen (PBMCs).

BDV zerstört die Wirtszellen nicht (kein zytopathogener Effekt), weder im Wirt (in vivo) noch in der Zellkultur (in vitro). Der primäre pathogene Effekt von BDV beruht auf einer funktionellen Störung in den infizierten Gehirnzellen, die vermutlich durch Interaktion mit Neurotransmitter-Rezeptoren induziert wird. Hinweise gibt es für eine (reversible) Blockade von Glutamat-Rezeptoren aus experimentellen Tierdaten. Der genaue Mechanismus und der Rezeptortyp sind noch unbekannt.

BDV-Infektionen sind beim Tier mit phasenhaften Verhaltensstörungen mit den Leitsymptomen Apathie, Aufmerksamkeitsverlust und Bewegungskoordinationsstörungen assoziiert. Beim Menschen ist nach Isolierung infektiöser humaner Bornaviren aus Patienten mit rekurrenten endogenen Affektstörungen eine Beteiligung an diesen und möglicherweise anderen cerebralen Funtkionsstörungen sehr wahrscheinlich (ebenfalls vermutlich über Neurotransmitterfunktionen). Endogene rekurrente Depressionen incl. der manisch-depressiven Form gehören mit 1-5% zu den bedeutenden psychiatrischen Erkrankungen und sind wegen des Schweregrads der Beeinträchtigung für die Betroffenen aber auch für die Gesellschaft aus sozioökonomischer Sicht ein beträchtliches Gesundheitsproblem.

BDV-Infektionen persistieren in Tier und Mensch, vermutlich lebenslang. Die Infektionsquellen sind noch gänzlich unbekannt. Der Infektionsverlauf zeichnet sich durch latente und aktivierte Phasen aus. Während der aktivierten Phasen kann es zu klinischer Symptomatik kommen. Bei Tieren (am besten untersucht sind Pferde) gibt es viele Infektionen ohne Krankheit (asympto-matische Träger). Die Anzahl kann bis zu 50% in einem Pferdebestand betragen, in dem eine Erkrankung registriert wurde. Das Risiko einer starken, mit Krankheit verbundenen Aktivierungsphase der Infektion hängt von genetischen Faktoren und der individuellen Belastung (Stressfaktoren, Immunsuppression) ab.

Beim Menschen gibt es wahrscheinlich kein generelles Erkrankungsrisiko für gesunde infizierte Personen, die keine Veranlagung zu einer Affektstörung haben. Dagegen ist ein erhöhtes Erkrankungsrisiko durch BDV-Infektion im engeren Sinne gegeben bei Personen mit einer angeborenen Veranlagung zur Entwicklung einer Affektstörung, mit einer bereits manifesten Affektstörung und bei deren noch gesunden Verwandten ersten Grades.

Es besteht ein unbestreitbarer, zunehmender Bedarf an zuverlässigen Diagnosesystemen zur Erfassung der BDV-Infektion, sowohl bei asymptomatischen Trägern (aus epidemiologischen Gründen) als auch zur Kontrolle der Infektionsphasen bei Erkrankten.

Da das BDV-Virus schon gut untersucht und bereits vollständig sequenziert ist, sind übliche Antikörpertests bekannt. Bis 1993 gab es nur die Möglichkeit eines Antikörpertests im Serum. Antikörper richten sich in der Regel, gegen das BDV-Nukleoprotein p40 (40kDa relatives Molekulargewicht) und das Phosphoprotein p24 (24kDa). Diese Antikörper neutralisieren das Virus nicht. Es stellte sich bald heraus, daß das Fehlen von spezifischen Antikörpern eine BDV-Infektion nicht ausschloß. Das bedeutet, daß die Antikörper nicht (oder nicht sehr lange) im Serum des Infizierten persistieren. Außerdem sind die bei natürlichen Infektionen bei Mensch und Tier erreichten AK-Konzentrationen (Titer) ohnehin nur gering und mit insensitiveren Testsystemen nicht immer erfaßbar.

Ein wichtiger Fortschritt, besonders für die Beurteilung krankheitsrelevanter Aktivierungen der BDV-Infektion, konnte durch die Entdeckung gemacht werden, daß PBMCs Virusproteine exprimieren, die man in den Zellen nach Aufschluß nachweisen kann. Diese Proteine werden gelegentlich auch im Plasma gefunden. Die Protein(AG)-Expression hat sich als der entscheidende Aktivitätsparameter erwiesen. Er korreliert gut mit klinischer Erkrankung (Mensch und Tier), ist quantitativ meßbar und ist sehr wichtig zur Einschätzung des weiteren Verlaufs (Genesungschancen, Ansprechen von Antidepressiva etc.). Die AG-Bestimmung kann auch nicht durch den Nachweis von Virusnukleinsäure über die Amplifikation mit nested-RT-PCR in den PBMCs ersetzt werden, da hierdurch lediglich das Virus selbst nachgewiesen wird, jedoch nichts über dessen derzeitige oder mögliche zukünftige Aktivität entnommen werden kann.

Der AG-Marker ist in aller Regel auf einen Teil (2-3 Wochen) der akuten Krankheitsphase beschränkt und in der Rekonvaleszenz nicht mehr nachweisbar. Die AK können im Anschluß je nach Stärke des Aktivierungsschubs intermittierend nachweisbar sein, sie können aber auch vollständig fehlen.

Dieser aktuelle Stand der BDV-Diagnostik versagt vor allem in der Rekonvaleszenz, in dem symptomfreien Intervall (das Jahre dauern kann) eines Patienten (Mensch und Tier) und bei infizierten Individuen ohne bisherige Erkrankung, d.h. zusammengefaßt bei BDV-Infektionen im Latenzzustand. Beide Testparameter, AG und AK, können in der Latenzphase falsch-negativ anzeigen bei nach wie vor bestehender Infektion.

In Journal of clinical Microbiology, 1997, Bd. 35, Nr. 7, S. 1661-1666, Horimoto, T. et al. (über MEDLINE AN 97339570) wird ein ELISA-Test zum Nachweis BDV-spezifischer Antikörper angegeben, bei dem Microwell-Platten mit BDV p40 antigen beschichtet wurden, um gegen dieses BDV-Nucleoprotein spezifische Antikörper abzufangen und nachzuweisen. Es wurde dort festgestellt, daß viele vorher auf andere Weise seropositiv getestete Proben ein negatives Ergebnis bezüglich der Antikörper ergaben. Hieraus wurde der Schluß gezogen, daß in den anderen Untersuchungen ein hoher Anteil falsch-positiver Ergebnisse vorhanden gewesen wäre. Aus den Ergebnissen ließe sich jedoch auch der Schluß ziehen, daß sich die BDV-Infektionen nicht in jedem Fall über den Antikörper-Titer nachweisen läßt. Ein vollständiges Diagnose-Konzept scheint daher erforderlich.

Die Aufgabe der Erfindung bestand daher darin, ein neues Nachweisverfahren für die Infektion zu entwickeln, um so eine möglichst lücklose Erfassung von BDV-Infektionen zu ermöglichen, die durch ihre Persistenz über sehr lange Zeiträume im Wechsel von akuten und latenten Phasen andauern können. Ebenfalls soll ein für einen solchen Nachweis verwendbarer diagnostischer Kit zur Verfügung gestellt werden.

Zur Lösung dieser Aufgabe ist erfindungsgemäß ein Verfahren zum Nachweis von Borna Disease Virus (BDV)-Infektion vorgesehen, bei welchem an einer zu untersuchenden Körperflüssigkeitsprobe in der Körperflüssigkeit frei zirkulierende Immunkomplexe aus BDV-Antigenen und hieran angelagerten spezifischen Antikörpern durch einen geeigneten immunologischen Test nachgewiesen werden. Die Erfindung basiert daher in einem wesentlichen Aspekt darauf, daß nachgewiesen werden konnte, daß in bestimmten Krankheitsphasen in Körperflüssigkeiten, z.B. im Blutserum, hohe Konzentrationen zirkulierender, aus BDV-Antigenen und vom Körper hierzu spezifisch gebildeten Antikörpern bestehender Immunkomplexe auftreten. Diese Immunkomplexe können insbesondere auch in Krankheitsphasen vorhanden sein, in denen Antigene und/oder Antikörper gerade nicht nachweisbar sind.

Grundsätzlich ist das Vorhandensein von Immunkomplexen, d.h. von im Blut frei beweglichen Komplexen aus Antigenen und Antikörpern, bereits bekannt, die Relevanz dieser Komlexe ist jedoch im Einzelfall sehr unterschiedlich.

Aus NYDEGGER, U.E. in: MASSEYEFF, R.F. [u.a.] [Hrsg.].: "Method of Immunological Analysis", VCH Verlagsgesellschaft, Weinheim [u.a.] 1993, Bd. 1, S 646-656 ist beispielsweise ein ELISA-Test für HIV-spezifische CICs bekannt. Die Autoren fanden hohe Level an zirkulierenden Immunkomplexen bei HIV-positiven Drogenabhängigen. Hierzu wird angegeben, daß etwa nur ein Zehntel des insgesamt vorhandenen Serum IgG's in CICs komplexiert war. Die Bedeutung der CICs ist demnach offenbar virusspezifisch. Bei BDV-Infektionen - wie auch teilweise bei HIV-Infektionen - kann der Anteil frei zirkulierender Immunkomplexe zeitweilig sehr hoch sein, so daß "freie" Antikörper und Antigene weitgehend darin gebunden sein können.

In Weiterbildung der Erfindung wird, basierend auf der vorstehenden Erkenntnis, ein völlig neues Diagnose-Konzept zur praktisch lückenlosen Erfassung persistenter BDV-Infektionen vorgestellt. Hierbei wird der neu entdeckte Parameter, der CIC, in Kombination mit bereits bekannten Parametern, nämlich Antigenen (AG) und Antikörpern (AK) bestimmt, was eine bisher nicht mögliche Erfassung verschiedener Phasen der persistenten BDV-Infektion ermöglicht. Die hierbei zusätzlich bestimmten Antigene sind vorzugsweise das BDV-Nukleoprotein p40 (40 kDa relatives Molekulargewicht), auch als "N-Protein" bezeichnet und das BDV-Phosphoprotein p24 (24 kDa relatives Molekulargewicht), auch als "P-Protein" bezeichnet. Das kombinierte Verfahren sieht ein komplettes Testsystem vor, bei dem eine Testkombination, bestehend aus der Bestimmung von BDV-spezifischen zirkulierenden Immunkomplexen (CIC) im Blutplasma, BDV-spezifischen Proteinen (Antigenen, AG) in weißen Blutzellen (PBMCs) sowie im Plasma und/oder BDV-spezifischen Antikörpern (AK) im Blutplasma durchgeführt wird.

Vorzugsweise ist die zu untersuchende Körperflüssigkeitsprobe eine Blutprobe, wobei alle 3 Parameter mit einer einzigen Blutprobe von ca. 10 ml Citratblut bestimmt werden können.

Die Blut-, und oder Liquorprobe kann folgendermaßen behandelt werden:
(a) aus der Blutprobe wird eine Blutplasmafraktion isoliert und an der Blutplasma-, Liquor- oder Urinprobe werden unabhängig voneinander folgende Tests durchgeführt:
   (1) ein Antigen-Nachweis auf BDV-Antigene,
   (2) ein Antikörpernachweis auf BDV-spezifische Antikörper,
   (3) ein CIC-Nachweis, vorzugsweise gemäß einem der Ansprüche 5 bis 9;
(b) aus der Blutprobe werden eine Blutplasmafraktion und eine Leukozytenfraktion präpariert und der Test gemäß (a)(1) wird im Falle einer Blutprobe an der Leukozytenfraktion und/oder der Blutplasmafraktion durchgeführt.

Durch die Erfindung konnte erstmals anhand zahlreicher Tests gezeigt werden, daß den CICs für die Diagnostik, aber auch zum Verständnis des Infektionsablaufs eine Schlüsselrolle zukommt. Wie gefunden wurde, persistieren CICs erheblich länger als lösliche, mit AK-Tests erfaßbare BDV-Antikörper und länger als das BDV-AG. CICs sind nach längeren symptomfreien Phasen immer noch nachweisbar, wenn AK-Test und AG-Test negativ ausfallen und lassen den Rückschluß auf zurückliegende Aktivierungsphasen mit AG-Freisetzung und dieses AG-komplexierende AK zu.

Durch die Einführung der CICs als diagnostischen Testparameter, der ggf. in Kombination mit AK und AG verwendet wird, schließt die Erfindung eine diagnostische Lücke, die zuvor nicht überbrückt werden konnte.

Die prinzipielle Existenz zirkulierender Immunkomplexe, d.h. frei beweglicher AG-AK-Komplexe ist für verschiedene Systeme bekannt. Die freie Komplexierung von AGs mit AKs im Serum und umgekehrt wurde auch bereits für verschiedene immunologische Tests ausgenutzt. Die Existenz von CICs bei BDV-Infektionen, insbesondere die lang anhaltende hohe Konzentration der CICs auch im Latenzintervall und die damit verbundene diagnostische Relevanz der CICs, war bisher gänzlich unbekannt.

Bei einigen Patienten mit endogenen Affektstörungen und BDV-Infektionen sind bei wöchentlichen Kontrollen auch in der akuten Phase ausschließlich extrem hohe CIC-Konzentrationen, aber keine freien AK und kein AG in PBMCs meßbar. Diese Patienten würden bei der herkömmlichen Diagnostik überhaupt nicht erfaßt. Bei diesem Infektionsverlauf muß vermutet werden, daß das in den PBMCs gebildete BDV-AG, das in hoher Konzentration gebildet wird, sofort ins Plasma übertritt und von den vorhandenen AK in CICs gebunden wird.

Die in der Erfindung beschriebene Bestimmung der CICs und insbesondere die Testkombination aus CIC-, AG- und AK-Bestimmung wird daher der persistenten BDV-Infektion mit ihrem zwischen Latenz und Aktivierung flukturierenden Verlauf erstmalig gerecht und hat damit große Bedeutung sowohl für die diagnostische Betreuung erkrankter Individuen als auch für die Erfassung latenter Infektionen bei Gesunden.

Bei Risikopatienten erlaubt die obligate Bestimmung der CICs eine nicht mehr auf die akute Erkrankung beschränkte BDV-Diagnostik. Die Untersuchung latenter Infektionen bei Gesunden war wegen der geringen Virusaktivität theoretisch nur in engmaschigen Kontrollen möglich, in der Praxis aber kaum machbar. Mit dem CIC-Test und der Testkombination können latente Infektionen gesunder aufgespürt werden, was von epidemiologischer Tragweite ist, da die Infektionsquellen und die Übertragungsmechanismen bei Mensch und Tier noch ungeklärt sind.

Die Verwendung der erfindungsgemäßen Verfahren ist für die Erfassung von BDV-Infektionen bei Menschen und Tieren geeignet und unabhängig von der klinischen Erkrankung möglich. Mit dem erfindungsgemäßen Nachweisverfahren können BDV-spezifische CICs für den Menschen und verschiedene Tierspezies quantifiziert werden.

Der Begriff "Nachweis" wird nach der Terminologie dieser Erfindung immer so verwendet, daß sowohl ein erster qualitativer Nachweis, wie auch eine quantitative Bestimmung der relativen CIC-Konzentrationen in Form eines Titers möglich ist.

Als ein geeigneter immunologischer Test bzw. ein geeignetes Verfahren zum Nachweis in Körperflüssigkeit zirkulierender Immunkomplexe wird erfindungsgemäß ein Verfahren mit den folgenden Schritten vorgeschlagen:
(1) Fixieren von monoklonalen oder polykonalen Antikörpern, die an die in den CICs enthaltenen Antigene spezifisch binden, über die Fc-Region in geeigneter Weise auf einem ggf. hierfür vorbereiteten Träger;
(2) Inkontaktbringen einer Körperflüssigkeits-, vorzugsweise einer Blutplasmaprobe, die auf Anwesenheit von CICs getestet werden soll, mit den Antikörpern;
(3) Inkontaktbringen eines Sekundärantikörpers einer anderen als der getesteten Spezies, vorzugsweise eines Ziege-anti-Spezies-Antikörpers, der spezifisch ist auf Antikörper der Spezies, deren Körperflüssigkeitsprobe verwendet wurde, mit der nach Schritten (1) und (2) behandelten Probe;
(4) Nachweis und/oder Bestimmung der Menge des Sekundärantikörpers durch ein geeignetes immunologisches Nachweisverfahren.

Hierbei sind die in Schritt (1) verwendeten BDV-spezifischen Antikörper vorzugsweise monoklonale BDV-spezifische N- und/oder P-Protein-Antikörper, die beispielsweise von der Maus gewonnen wurden.

Der ggf. in geeigneter Weise vorbereitete Träger kann eine absorptiv fixierende Kunststoff-Testplatte oder ein entsprechendes Teströhrchen sein, wobei diese Platte oder das Röhrchen vorzugsweise zunächst mit Sekundärantikörpern möglichst vollständig belegt wird, die gegen die Spezies, von der die CIC-Antigen-spezi-fifischen Antikörper gewonnen wurden, spezifisch sind, und in einem hierauf folgenden Schritt der Plattenvorbereitung die CIC-Antigen-spezifischen Antikörper auf diese Sekundärantikörperschicht aufgebracht werden.

Alternativ kann die Fixierung der CIC-Antigen-spezifischen Antikörper in Schritt (1) des Verfahrens auch mit einem geeigneten anderen Verfahren erfolgen, mit dem es möglich ist, die Antikörper in geeigneter Ausrichtung sicher auf der Platte zu fixieren, z.B. durch Aufbringen einer Grundschicht aus C1q auf einen Polystyrolträger. Das Aufbringen einer solchen Grundschicht bewirkt generell, daß mit den kostbareren CIC-spezifischen Antikörpern sparsamer umgegangen werden kann.

Der Nachweis gemäß Schritt (4) des Verfahrens kann beispielsweise über einen enzymgekoppelten Sekundärantikörper erfolgen, an dem mit einem geeigneten Substrat eine Farbreaktion ausgelöst wird. In der derzeit bevorzugten Ausführungsform wird der Sekundärantikörper mit alkalischer Phosphatase gekoppelt und mit p-Nitrophenylphosphat visuell sichtbar bzw. mit optischen Detektoren auslesbar gemacht, indem die Reaktion zwischen der alkalischen Phosphatase und dem para-Nitrophenyl-phosphat, die zu einem gelben Farbstoff führt, ausgenutzt wird.

Als Sekundärantikörper im Sinne der Erfindung wird ein Antikörper bezeichnet, der nicht für ein Antigen spezifisch ist, sondern für einen anderen Antikörper, d.h. einen als "fremd" erkannten Antikörper einer anderen Spezies.

Die Bestimmung der CICs kann in jeder Körperflüssigkeit erfolgen, in der diese CICs in hinlänglicher Konzentration vorkommen. In der derzeitig bevorzugten Ausführungsform ist die Körperflüssigkeit, an der der Test vorzugsweise vorgenommen wird, eine aus Citratblut gewonnene Blutplasmaprobe. Es kann jedoch u.a. auch Liquor verwendet werden.

Für die Testkombination werden BDV-Antigene und BDV-spezifische Antikörper benötigt. Als Antigen-Quelle können mit BDV infizierte Zellen dienen. Der Test wird nicht gestört oder verfälscht, wenn anstelle isolierter Antigene (homogenisierte) antigenhaltige Zellsuspensionen direkt verwendet werden. In einem in den Beispielen zitierten Test (siehe "AK-Test") wurde z.B. eine 10%ige Gehirnsuspension eines an der Borna'schen Krankheit verstorbenen Pferdes in Verdünnung verwendet.

Der Anmelder hat bei der Deutschen Sammlung für Mikroorganismen und Zellkulturen fetale menschliche Oligodendrogliazellen fremdviren- und mykoplasmenfrei hinterlegt, die als Zellinie wachsen und persistent mit BDV infiziert sind. Diese Hinterlegung erfolgte nach den Bedingungen des Budapester Vertrags am 12. 12. 1997; der Kultur OLIGO/TL wurde die Bezeichnung und Identifikationsnummer "DSM ACC 2334" zugewiesen.
Die Zellinie stammt aus dem Bestand des Anmelders. Die fetalen menschlichen OL-Zellen sind ohne CPE persistent mit BDV infiziert. Die interne Bezeichnung dieser Zellinie, die etwa 110 Passagen durchlaufen hat, ist OLIGO/TL (bzw. OL/TL).
Aus diesen Zellen kann jederzeit Antigen, das für die Verwendung gemäß der Erfindung geeignet ist, gewonnen werden. Außerdem können mit Hilfe dieser Zellinie andere Zellinien infiziert werden - beispielsweise auch Tierzellinien. Der Titer beträgt ca. 10³ FFU/ml. Insbesondere enthalten die Zellen auch die oben beschriebenen Antigene p40 und p24.

Mit Hilfe der Zellsuspension als solcher oder auch mit daraus gereinigten Proteinen können jederzeit BDV-spezifische monoklonale oder polyklonale Antikörper in an sich bekannter Weise produziert werden. Derzeitig bevorzugt werden monoklonale N- und P-Antikörper von der Maus. Dazu werden, wie üblich, Mäuse infiziert bzw. immunisiert und aus deren B-Zellen Hybridomakulturen hergestellt. Die Überstände werden auf Antikörper durchgetestet (screening), die BDV-spezifisch "anfärben". Bezüglich der Gewinnung von Antikörpern siehe auch "H. Ludwig et al., Arch. Virol. (1993) Suppl. 7:111-113".

Die Erfindung umfasst weiterhin einen diagnostischen Kit zum Nachweis von BDV-Infektionen. Dieser Kit bzw. dieses Test- oder Nachweissystem enthält BDV-spezifische monoklonale oder polyklonale Antikörper, Mittel zum Inkontaktbringen dieser Antikörper mit einer Probe, von der vermutet wird, dass sie BDV-CICs enthält, sowie Mittel zum Nachweis der angelagerten Antigen-Antikörper-Komplexe (CICs), und zwar insbesondere Sekundärantikörper einer anderen als der getesteten Spezies, vorzugsweise Ziege-anti-Spezies-Antikörper, die spezifisch sind auf Antikörper der Spezies, deren Körperflüssigkeitsprobe verwendet wurde. Alternativ enthält der Kit mit BDV-Antigen belegte BDV-spezifische monoklonale oder polyklonale Antikörper und Mittel zum Inkontaktbringen dieser Antigen-belegten Antikörper mit einer Probe, falls von dieser vermutet wird, dass sie BDV-Antikörper enthält, sowie Mittel zum Nachweis der dann angelagerten BDV-Antikörper, wie oben beschrieben.

Der diagnostische Kit kann ferner eine Einheit umfassen, auf oder in der die BDV-spezifischen Antikörper immobilisiert vorliegen. Eine solche Einheit kann eine in bestimmter Weise vorbereitete Testplatte oder ein innen beschichtetes Teströhrchen sein.

Bei einem bevorzugten Immobilisierungsverfahren sind die BDV-spezifischen Antikörper monoklonale oder polyklonale, von einer Spezies I gewonnene Antikörper, die dadurch immobilisiert sind, dass sie auf einen mit einem von einer anderen Spezies (Spezies 11) gewonnenen Spezies II-anti-Spezies I IgG-beschichteten Träger, vorzugsweise in Form einer Testplatte oder eines Teströhrchens, aufgebracht und hierdurch fixiert sind.

Weiter vorzugsweise sind die Antikörper der Spezies I polyklonale oder monoklonale Mausantikörper, vorzugsweise P-Protein- und/oder N-Proteinspezifische monoklonale Mausantikörper, wobei die adsorptive Beschichtung des Trägers aus einem Anti-Maus-IgG, vorzugsweise einem Ziege-Anti-Maus-IgG besteht. Alternativ können die BDV-spezifischen Antikörper auf oder in der Einheit auch über Polystyrol gebundenes C1q fixiert bzw. mit einer anderen geeigneten Methode immobilisiert sein.

Die Erfindung umfasst ferner die Verwendung eines BDV-Antigengemisches, gewonnen aus Körperflüssigkeit und/oder Zellhomogenisat mit BDV erkrankter Tiere oder Menschen, das zumindest in seiner qualitativen Zusammensetzung einem Antigengemische entspricht, welches mit Hilfe der unter der Nr DSM ACC 2334 hinterlegten Zellkultur gewonnen werden kann, zur Herstellung von Antikörpern für einen Kit gemäß einem der Ansprüche 10 bis 16 oder die Verwendung in einem Verfahren gemäß einem der Ansprüche 1 bis 9 und die Verwendung von Antikörpern, hergestellt gemäß Anspruch 17, in einem Verfahren gemäß einem der Ansprüche 1 bis 9.

### Beispiele

Um das Verständnis der Erfindung zu erleichtern werden im folgenden die technischen Einzelheiten der Testkombination beschrieben, von der Behandlung der Blutprobe bis zur Auswertung. Die Anwendung wird mit einzelnen Beispielen illustriert. Die Beispiele dienen rein illustrativen Zwecken. Selbstverständlich können die Prinzipien der Erfindung auch auf andere Weise umgesetzt werden.

### Teil I: Aufbereitung des zu testenden Probenmaterials

Für die Untersuchung auf eine BDV-Infektion werden 10 ml Citratblut benötigt. Dabei werden zu 1 ml einer 0,106 molaren Natriumcitrat-2-hydrat-Lösung 9 ml venösen Probandenblut gegeben und gut gemischt. Empfehlenswert ist die Verwendung von fertigen Citratröhrchen, z.B. die 10 ml Monovette 9NC von Sarstedt. Die Probe soll bis zum Versand an das Untersuchungslaboratorium bei 4 °C gelagert werden. Ein Versand per Eilzustellung ohne Kühlung (Dauer 1 bis maximal 3 Tage) beeinträchtigt die Probenqualität nicht.

Die Citratblutprobe wird mit einer Dichtegradienten-Zentrifugation in die Plasmafraktion und die zellulären Blutbestandteile getrennt. Die Plasmafraktion wird zur Testung von CIC, AG und AK verwendet. Die Zellfraktion der weißen Blutzellen (PBMCs) wird nach Aufbereitung zur AG-Testung verwendet. Wenn nur auf CICs getestet werden soll oder nur Plasmatests durchgeführt werden kann die Zellfraktion verworfen werden.

Für die Trennung der Bestandteile wird je nach Tierspezies eine Ficoll-Trennlösung (Biochrom) unterschiedlicher Dichte verwendet.

Für 6 ml Blut werden benötigt:

| | |
|---|---|
| Mensch | 3 ml Ficoll mit Dichte 1,077 |
| Pferd | 3 ml Ficoll mit Dichte 1,090 |
| Rind | 2 ml Ficoll mit Dichte 1,077 + 1 ml Ficoll mit Dichte 1,068 |
| Katze | 2,8 ml Ficoll mit Dichte 1,077 + 0,2 ml PBS pH 7,2 |
| Hund | 2,8 ml Ficoll mit Dichte 1,077 + 0,2 ml PBS pH 7,2 |
| Kaninchen | 2,8 ml Ficoll mit Dichte 1,077 + 0,2 ml PBS ph 7,2 |

### I. Protokoll zur Trennung einer 10 ml Citratblutprobe

(1) Konische 10 ml-Einwegröhrchen mit 3 ml Ficoll-Trennlösung der entsprechenden Dichte füllen und mit maximal 6 ml Citratblut überschichten
(2) Probe für 20 min bei 1049 g (2200 Upm, Heraeus Christ Minifuge) zentrifugieren (für alle Proben mit Ficoll < 1,090 Dichte); bei Ficoll mit 1,090 Dichte für 20 min bei 1249 g (2400 Upm) zentrifugieren
(3) Plasma-Fraktion (Überstand) vorsichtig mit Einweg-Pasteurpipette abnehmen; für die Soforttestung am selben Tag bei 4°C lagern, für eine spätere Testung bei -20°C lagern
(4) Die Leukozytenfraktion bildet einen erkennbaren Ring auf bzw., über dem Ficoll, während die (nicht benötigte) Erythrozytenfraktion im Ficoll als Pellet vorliegt. Nach dem Abnehmen des Plasmas, Zellring mit Einwegpasteurpipette aufnehmen und in ein neues konisches 10ml-Plastikröhrchen überführen, mit PBS auffüllen und gut mischen
(5) Weiße Blutzellen (PBMCs) für 10 min bei 1952 g (3000 Upm) zentrifugieren. Überstand verwerfen und Pellet in maximal 0,5 ml PBS = Konzentrierung ca. 10 f aufnehmen. Lösung in sterile Tiefgefrierröhrchen (z.B. Nunc, 1,5 ml) mit Schraubverschluß überführen.
(6) Für die sofortige Testung interner BDV-Antigene (s. unten), Lagerung der Zellen in PBS bei 4°C, bei späterer Testung Lagerung bei mindestens -20°C, besser bei -70°C.

### II. Testsysteme

Für die Testung von AG in PBMCs und/oder im Blutplasma und CIC sowie AK im Plasma wird eine Testkombination von EIA (enzyme immune assay) - Basis (Festphasen-Assay) verwendet. Die ersten beiden Testschritte sind für alle drei Testsysteme gleich. Die nachfolgende Beschreibung in Form von Testprotokollen teilt sich daher auf in:
- (A): Beschichtung der Testplatten (A1 und A2)
- (B, C, D): Protokoll für AG, CIC und AK Test.

### Protokoll für EIA (Doppel-sandwich-Typ)

### (A) Protokoll zur Beschichtung der EIA-Platten

Es werden als Plastikträger für die nachfolgenden Reaktionen Maxi Sorp Immuno Module (Nunc® Maxi Sorp F8, Cat.No.469949) verwendet. In einen Rahmen passen 12 Module mit 8 senkrecht angeordneten flachen Plastikvertiefungen hinein und haben exakt die Maße einer 96-Loch Mikrotiterplatte (Standardmaß für EIA-Systeme):

(A1) 0,1 ml pro Vertiefung von AffiniPure® Ziege-anti-Maus IgG, Fc-Fragment, absorbiert gegen Human, Rind, Pferd Serumproteine (Dianova®, Kat.Nr. 115-005-071), verdünnt 1:1000 (= 1,8 Mikrogramm Protein pro Milliliter) in Kopplungspuffer; Inkubation über Nacht bei 4°C oder 2 h bei 37°.

### Waschen 3x in Waschpuffer (Dynatech 96-Loch Platten-Washer)

(A2) 0,1 ml pro Vertiefung von monoklonalen Antikörpern gegen das BDV-N-Protein p40 und das BDV-P-Protein p24 (W1 und KFU2, ref.: Arch. Virol. (1993) Suppl. 7:111-133) (als "Catch"-Antikörper), jeweils verdünnt 1:500 in Verdünnungspuffer. Inkubation für 2 h bei 37°C oder über Nacht bei 4°C.

Die Platten können danach bei 4°C mindestens bis zu 4 Wochen vor Weiterbenutzung gelagert werden.

### (B) Protokoll für Bestimmung von BDV-Antigenen (AG) in PBMCs:

### Vorbereitung:

- Waschen der bis A2 beschichteten Platten, 3x in Waschpuffer
- Ultraschall-Behandlung (20 Zyklen min⁻¹, 40mA) (Branson Sonifier B15P; ref. Mol. Psychiatry (1996) 1:200-212) der in I. isolierten PBMCs.

### AG-Test:

(B1) Probenverdünnung: 0,1 ml Verdünnungspuffer in jede Vertiefung der vorbeschichteten Platten vorlegen, in A ultrabeschallte PBMCs (0,1 ml) oder Blutplasma (0,1 ml) zugeben (=1:2) und weitere 7 Stufen zur Basis 2 (bis 1:256) weiterverdünnen; Inkubation über Nacht bei 4°C.

### Waschen 3x in Waschpuffer

(B2) 0,1 ml pro Vertiefung BDV-spezifisches Kaninchen Antiserum (z.B. GC 12, Immunfluoreszenztiter 1: 10000) (= Detektions-Antikörper) in entsprechender Verdünnung (1:1000, 10 fach konzentrierter als IF-Titer) in Verdünnungspuffer; Inkubation 2 h bei 37°C.

### Waschen 3x in Waschpuffer

(B3) 0,1 ml pro Vertiefung von mit alkalischer Phosphatase gekoppeltem Affini Pure® Ziege-anti-Kaninchen IgG, Fc-Fragment-spezifisch, absorbiert gegen Human Serumproteine (Dianova®, Kat. Nr. 111-055-046) (= Sekundär-Antikörper), verdünnt 1:3000 in Konjugatverdünnungspuffer; Inkubation für 1 h bei 37°C.

### Waschen 3x in Waschpuffer

(B4) 0,1 ml pro Vertiefung der Substratlösung (1mg/ml p-Nitrophenylphosphat ; Sigma, erhältlich als Substrat-Tabletten) in Substratverdünnungspuffer; Inkubation 5-10 min bei Raumtemperatur, bzw. bis zur Entwicklung der Farbreaktion (Umschlag von farblos nach gelb) in der Positivkontrolle.

(B5) 0,05 ml pro Vertiefung der Stoplösung (3 molare NaOH-Lösung) zugeben

(B6) Messung bei 405 nm in einem Multiscan für Mikrotiterplatten (z.B. BIO RAD 2550 EIA READER oder Dynatech); "Blank"-Messung gegen nicht umgesetztes Substrat. Semiquantitative Auswertung ausreichend: (+) = schwach positiv bis maximal 4+ = sehr stark positiv; +/- bedeutet grenzwertig und erfordert eine zweite Untersuchung. (Beispiel s. bei Anwendung).

### (C) Protokoll für die Bestimmung von BDV-spezifischen zirkulierenden Immunkomplexen (CIC) im Blutplasma:

### Vorbereitung:

- Waschen der bis A2 beschichteten Platten, 3x in Waschpuffer

### CIC-Test:

(C1) Probenverdünnung: 0,1 ml Verdünnungspuffer in jede Vertiefung der vorbeschichteten Platten vorlegen; in A: 0,09 ml Verdünnungspuffer zufügen + 0,01 ml des in I. präparierten Blutplasmas (= Verdünnung 1:20), weitere 7 Stufen zur Basis 2 (bis 1:2560) weiterverdünnen; Inkubation für 1-2 h bei 37°C (Standardzeit: 1h bei 37°C).

### Waschen 3x mit Waschpuffer

(C2) 0,1 ml pro Vertiefung von mit alkalischer Phosphatase gekoppeltem Ziege-anti-Spezies (entsprechend der Spezies der Probe) IgG, Fc-Fragment (ebenfalls erhältlich von Dianova®) (=Sekundär-Antikörper), verdünnt 1:3000 in Konjugatverdünnungspuffer; Inkubation für 1 h bei 37°C.

### Waschen 3x in Waschpuffer

(C3) 0,1 ml pro Vertiefung der Substratlösung (1 mg/ml p-Nitrophenylphosphat; Sigma, erhältlich als Substrat-Tabletten) in Substratverdünnungspuffer; Inkubation 5-10 min bei Raumtemperatur, bzw. bis zur Entwicklung der Farbreaktion (Umschlag von farblos nach gelb) in der Positivkontrolle

(C4) 0,05 ml pro Vertiefung der Stoplösung (3 molare NaOH-Lösung) zugeben.

(C5) Messung bei 405 nm in einem Multiscan für Mikrotiterplatten (z.B. BIO RAD oder Dynatech); "Blank"-Messung gegen nicht umgesetztes Substrat. Semiquantitative Auswertung ausreichend von (+) bis 4+ (vgl. auch Test B); Titerangaben mit Endpunktbestimmung aber ebenfalls möglich. (Beispiele s. Anwendung).

### (D) Protokoll für die Bestimmung von BDV-spezifischen Antikörpern (AK) im Blutplasma:

### Vorbereitung:

- Waschen der bis A2 beschichteten Platten, 3x in Waschpuffer

### AK-Test:

(D1) 0,1 ml pro Vertiefung von einer 10%igen Gehirnsuspension (= Detektions-Antigen) eines an der Borna'schen Krankheit verstorbenen Pferdes, in entsprechender Verdünnung (1:50) in Verdünnungspuffer; alternativ kann Zellkulturüberstand der am 12.12.1997 unter Nr DSM ACC2334 bei der DSMZ hinterlegten persistent mit einem Borna-Disease-Virusstamm infizierten Zellinie verdünnt zwischen 1:100 und 1:300 in Verdünnungspuffer,
verwendet werden (bei Virustiter 10⁶ ffu/ml: 1:300). Inkubation über Nacht bei 4°C.(ffu = focus forming units)

### Waschen 3x in Waschpuffer

(D2) Probenverdünnung: 0,1 ml Verdünnungspuffer in jede Vertiefung der vorbeschichteten Platten vorlegen, in A: 0,08 ml Verdünnungspuffer zufügen + 0,02 ml des in I. präparierten Blutplasmas, vorverdünnt 1:5, (= Verdünnung 1:50), weitere 7 Stufen zur Basis 2 (bis 1:6400) weiterverdünnen; Inkubation für 1 h bei 37°C.

### Waschen 3x mit Waschpuffer

(D3) 0,1 ml pro Vertiefung von mit alkalischer Phosphatase gekoppeltem Ziege-anti-Spezies (entsprechend der Spezies der Probe) IgG, Fc-Fragment-spezifisch (Dianova®) (= Sekundär-Antikörper), verdünnt 1:3000 in Konjugatverdünnungspuffer; Inkubation für 1 h bei 37°C.

### Waschen 3x in Waschpuffer

(D4) 0,1 ml pro Vertiefung der Substratlösung (1mg/ml p-Nitrophenylphosphat; Sigma, erhältlich als Substrat-Tabletten) in Substratverdünnungspuffer; Inkubation 5 - 10 min bei Raumtemperatur, bzw. bis zur Entwicklung der Farbreaktion (Umschlag von farblos nach gelb) in der Positivkontrolle.

(D5) 0,05 ml pro Vertiefung der Stoplösung (3 molare NaOH-Lösung) zugeben.

(D6) Messung bei 405 nm in einem Multiscan für Mikrotiterplatten (z.B. BIO RAD oder Dynatech); "Blank"-Messung gegen nicht umgesetztes Substrat. Auswertungsmöglichkeiten wie Test C.

### III. Pufferlösungen

### Kopplungspuffer:

Stammlösung A: 0,02 M NaH₂PO₄ • H₂O 2,76 g ad 1000 ml A.bidest
Stammlösung B: 0,02 M Na₂HPO₄ • 2H₂O 3,561 g ad 1000 ml
A.bidest

### Gebrauchslösung:

65 ml Lösung A + 45 ml Lösung B + 14,6 g NaCl ad 1000 ml
A.bidest pH 7,6.

### Waschpuffer:

| | | |
|---|---|---|
| NaCl | 45 g | (9 g pro 1000 ml) |
| Tween 20 | 2,5 g | (0,5 g pro 1000 ml) |
| NaN₃ | 1,0 g | (0,1g pro 1000 ml) |
| ad 5000 ml A. bidest | | |

### Verdünnungspuffer:

| | |
|---|---|
| KH₂PO₄ | 0,2 g |
| Na₂HPO₄ • 12 H₂O | 2,9 g |
| NaCl | 8,0 g |
| KCl | 0,2 g |
| Tween 20 | 0,5 g |
| NaN₃ | 0,2 g |
| ad 1000 ml A.bidest, pH 7,2 muß kontrolliert bzw. eingestellt werden. | |

### Konjugatverdünnungspuffer (TBS-TWEEN):

| | |
|---|---|
| TRIS | 2,4 g |
| NaCl | 8,0 g |
| KCl | 0,2 g |
| Tween 20 | 0,5 g |
| in 900 ml A.bidest lösen, mit konz. HCL auf pH 8,0 einstellen, dann auf 1000 ml auffüllen. | |

### Substratverdünnungspuffer (DIÄTHANOLAMINPUFFER):

| | |
|---|---|
| Diäthanolamin | 97 ml |
| MgCl₂ | 0,1 g |
| NaN₃ | 0,2 g |
| in 843 ml A.bidest lösen, dann 60 ml 1 N HCL zugeben, pH 9,8 einstellen. | |

### Stoplösung (3 M NaOH):

| | |
|---|---|
| NaOH | 120 g |
| od 1000 ml A.bidest | |

### IV. Bezugsquellen kommerziell erhältliche Reagenzien für die beschriebenen Testsysteme:

1. Ficoll®-Trennmedien: Biochrom KG, D-12247 Berlin
2. Citratröhrchen: Sarstedt, D-51588 Nümbrecht
3. EIA Mikrotiter-Strips (Maxi Sorp Immuno Module) F-Form: Nunc, Roskilde, Dänemark
4. Enzymkonjugate für Sekundär-Antikörper: Dianova, Hersteller: Jackson Immuno Research Labs Inc., West Grove, PA, USA
5. Waschgerät für EIA-Teste: Dynatech Ultrawash plus; Dynatech Labs. Inc. Chantilly, VA, USA
6. Substrat für Enzym-Konjugate (für alkalische Phosphatase): Sigma Chemical Co., St. Louis, MO, USA
7. Puffersubstanzen (in III) außer KCL: Merck KGaA, D-64271 Darmstadt, Fluka Chemie AG, CH-9471 Buchs
8. Photometer (für Mikrotiterplatten): BIO RAD Labs. Hercules, Ca, USA oder Dynatech Labs Ind., Chantilly, VA, USA
9. Ultraschallgerät zum Zellaufschluß der PBMCs: Branson Sonifier, B15JP, Branson Sonic Power Company, Danbury, CT, USA.

### V. BDV-spezifische Reagenzien

### 1. Monklonale Antikörper:

### benötigt als Basisbeschichtung (Schritt A2) für Tests B, C, D.

1a) W1, erkennt ein sequentielles Epitop auf dem N-Protein (p40) von BDV. Die Bindungsstelle ist konserviert, d.h. wird wirtsspezies-übergreifend erkannt. Der Antikörper ist in der Referenz Arch. Virol. (Suppl) (1993) 7:111-133 charakterisiert.

1b) KFU 2, erkennt ein sequentielles Epitop auf dem P-Protein (p24) von BDV. Die Bindungsstelle ist konserviert, d.h. wird wirtsspezies-übergreifend erkannt. Der Antikörper ist in der Referenz Arch. Virol. (Suppl) 7:111-133 (1993) charakterisiert.

### 2 Kaninchen-Antiseren (spezifische Detektionsantikörper): benötigt für Test B (AG) in Schritt B2.

Es können Seren experimentell infizierter Kaninchen verwendet werden (Ref. wie unter V, 1a). Sie müssen einen Mindesttiter von 1:2000 im Immunfluoreszenztest haben und im Western-Blot mindestens das N- und P-Protein von BDV (p40 und p24) erkennen. Da Bornaviren nach dem jetzigen Forschungsstand genetisch sehr stabil sind, d.h. nur geringe Sequenzunterschiede in verschiedenen Virusstämmen gefunden wurden, erkennen polyklonale Antiseren in der Regel die Proteine von Bornaviren verschiedener Spezies.

Dies gilt auch für die bisher erhaltenen wenigen Humanstämme (Ref. Mol. Psychiatry (1996) 1:200-212; Virus Res. (1996) 44: 33-44.), obwohl die Humanstämme auf Aminosäure-Ebene relevante Punktmutationen zeigen, die bei Tierstämmen bisher nicht gefunden wurden.

Zusätzlich zu dem in der Testbeschreibung genannten Serum GC12 und weiteren, durch Infektion mit Tierreferenzstamm V erhaltenen Antiseren können definierte Kaninchenseren verwendet werden, die durch experimentelle Infektion mit den verschiedenen humanen BDV-Stämmen erzeugt worden sind (Ref. Mol. Psychiatry (1996) 1:200-212) und das gesamte Spektrum viraler Proteine erkennen.

### 3. Definierte Antigen-Suspensionen benötigt für Test D (AK-Test) in Schritt D

Gehirnsuspensionen (10%ig) an der Borna'schen Krankheit verstorbener Pferde enthalten eine hohe Konzentration viraler Proteine (insbesondere N- und P-Protein). Eine als Folge der BDV-Infektion sich entwickelnde progrediente neurologische Erkrankung mit Todesfolge ist selten im Vergleich zu phasisch auftretenden, spontan remittierenden Verhaltensstörungen bzw. zu subklinisch verlaufenden BDV-Infektionen (Ref.: Arch. Virol (1997) Suppl. 13: 167-182). Die als sog. klassische Borna'sche Krankheit bezeichnete Enzephalitis wird wahrscheinlich durch eine außer Kontrolle geratene Virusproduktion verursacht. Die Virustiter im Gehirn erreichen 10⁵ bis 10⁶ FFU/ml und liegen damit im Bereich experimentell infizierter Labortiere (Ref.: Prog. med. Virol. (1988) 35: 107-151).

Als Antigen-Suspensionen können direkt definierte Gehirnsuspensionen eingesetzt werden, zu denen der Virus- und Antigentiter bestimmt wurde. Für den AK-Test in dem beschriebenen Aufbau sind auch Suspensionen aus persistent mit BDV (Tier- und Menschenstämme) infizierten Zell-Linien verwendbar. Letztere eignen sich besonders für Standardisierungszwecke. Verwendbare Zell-Linien sind gemäß dem Budapester Vertrag unter Nummer DSM ACC 2334 bei der DSMZ hinterlegt (Hinterlegungsdatum 12.12.97).

Definierte Antigen-Lösungen sind für Test B (AG-Test) als Positivkontrolle einsetzbar.

### VI. Kurzbeschreibung der Testprinzipien der in der Erfindung beschriebenen Testkombination

Die Testkombination ist als Tripeltest für drei verschiedene infektionsrelevante BDV-Testparameter, nämlich Antigen (AG, Test B), zirkulierende Immunkomplexe (CIC, Test C) und Antikörper (AK, Test D) in einer einzigen Blutprobe konzipiert. Die Tests können auch jeweils einzeln durchgeführt werden, beispielsweise zur Überprüfung ganz bestimmter vermuteter Krankheitsphasen. Die Teste sind als Festphasenassay aufgebaut und werden photometrisch ausgewertet. Die Testreaktionen und Abläufe finden in Mikrotitersystemen statt, die heute Laborstandard sind.

Der als Basisbeschichtung beschriebene **Teil A** wird für alle drei bzw. vier Teste gleichermaßen genutzt. Die Basisbeschichtung ist ein Kernstück der Testkombination und ist für die hohe BDV-Spezifität verantwortlich. Diese wird durch ein Set aus zwei monoklonalen Antikörpern erzeugt, die gegen konservierte Epitope des N- und P-Protein von Bornavirus (p40, p24) gerichtet sind. Ein sehr effizienter Einsatz dieser "Catch"-Antikörper wird durch eine Vorbeschichtung mit einem Anti-Maus-Antikörper erzielt. Ohne diese Vorbeschichtung ist eine affinitätschromatographische Reinigung der spezifischen monoklonalen Antikörper notwendig. **Im Test B für den AG-Nachweis** werden in den PBMCs oder im Blutplasma (frei) vorhandene p40 und p24 Proteine von den "Catch"-Antikörpern gebunden. Mit polyklonalen Detektions-Antikörpern vom Kaninchen, die ebenfalls BDV-spezifisch sind, wird das gebundene Antigen aus der Probe erkannt und über das Enzym-gekoppelte Indikatorsystem sichtbar gemacht. Dieses sogenannte "double-Sandwich"-Prinzip hat den Vorteil, daß geringe Mengen von BDV-Antigen in einer großen Menge zellulärer Proteine, durch spezifische Bindung an zwei verschiedene Antikörpersysteme, nachweisbar werden.

Im Test C für den CIC-Nachweis wird der Antigenteil von im Plasma vorhandenen BDV-Immunkomplexen von den "Catch"-Antikörpern gebunden. Der Antikörperteil des Immunkomplexes wird in dem nächsten Reaktionsschritt mit einem Enzymmarkierten Anti-Antikörper (Sekundär-Antikörper) gebunden und dies über das Indikatorsystem sichtbar gemacht. Mit diesem in der Praxis verblüffend einfachen Testaufbau werden nur CICs sichtbar, die BDV-spezifisch und damit diagnostisch relevant sind. Dieser Test ist das Kernstück der Erfindung und schließt die bisher vorhandene diagnostische Lücke. BDV-spezifische CICs wurden von den Erfindern entdeckt und ihr Nachweis entwickelt.

**Im Test D für den AK-Nachweis** werden im Plasma vorhandene (freie) BDV-Antikörper gegen p40 und p24 über eine Detektions-Antigenlösung bestimmt, die zuvor von den "Catch"-Antikörpern gebunden wurde. Die spezifisch gebundenen Antikörper werden über Enzym-markierte Anti-Antikörper (Sekundärantikörper), das Indikatorsystem, sichtbar gemacht. Der Testaufbau hat den Vorteil, daß als Detektions-Antigenlösung ungereinigte infizierte Gehirn- oder Zellkultursuspensionen verwendet werden können, ohne an Spezifität zu verlieren. Die Spezifität wird durch die "Catch"-Antikörper gewährleistet.

Für andere einfachere AK-Tests auf EIA-Basais wird immer gereinigte Antigenlösung erforderlich sein, die als Festphase an den Plastikträger gebunden werden muß. Diese Teste sind zwar kürzer in der Durchführung, aber durch die Antigenreinigung aufwendig und kostspielig.

Als Standard-BDV-Antikörpertest wird anderenorts gegenwärtig der indirekte Immunofluoreszenztest durchgeführt ( Ref.: J. Med. Virol. (1992) 36:309-315). Der Test hat gegenüber neuen Systemen den Vorteil, daß die Ergebnisse quantitativ und qualitativ mit anderen Laboratorien verglichen werden können (Review: CTMI (1995) 190:103-130). Immunfluoreszenztiter sind in der Regel niedriger als EIA-Titer. In den in Sektion VII dargestellten Längsschnittuntersuchungen wurden die Antikörper mit Immunfluoreszenz bestimmt, um Vergleichbarkeit zu gewährleisten mit nur auf serologischer Basis erhobenen Ergebnissen anderer.

Der in Testkombination dargestellte AK-Test auf EIA-Basis verbessert die Nachweismöglichkeit der Antikörper wegen höhere Sensivität, ändert aber im Endergebnis nichts an den in der Einführung beschriebenen niedrigen Titern und dem Absinken unter die Nachweisgrenze. Deswegen schließt ein fehlender AK-Nachweis mit herkömmlichen Methoden eine BDV-Infektion nicht aus. Das nur vorübergehende Auftreten freier Antikörper ist durch die CIC-Bildung erklärbar.

Aufgrund der vier Testparameter ergibt sich folgendes Diagnosekonzept:

**cAG:** Der Antigentest aus PBMCs ist geeignet als Akutmarker für die Virusvermehrung. cAG ist nur kurz während der Krankheitsphase meßbar.

**pAG:** Das ins Plasma exprimierte Antigen tritt als Ergebnis der Virusvermehrung in den Zellen auf. Es erscheint im allgemeinen etwas später als cAG, manchmal aber auch mit diesem gleichzeitig, und ist oft einige Wochen nachweisbar. pAG ist ebenfalls ein Akutmarker während der Krankheitsphase. Seine Nachweisbarkeit ist jedoch von der Schnelligkeit der Antikörper- und anschließenden CIC-Bildung abhängig. Bei Verlaufsuntersuchungen (wöchentlich) wurde festgestellt, daß bei einzelnen Proben nur pAG positiv gemessen wird (z.B. Woche 1 ; nur pAG, Woche 2: pAG + CIC etc.).

**CIC:** Die zirkulierenden Immunkomplexe sind sehr gute Infektionsmarker und Therapie-Kontrollmarker. Unter antiviraler Therapie ist langfristig ein Verschwinden der CICs bzw. keine Neubildung zu beobachten. Die Halbwertzeit der CICs beträgt theoretisch vier Wochen; CICs sind jedoch oft viele Wochen nach dem akuten Krankheitsgeschehen noch nachweisbar. Ohne Therapie bleibt ein wechselnder Level an CICs i.d.R. erhalten. Die Messung der CICs it als Screening latenter Infektionen daher auch bei Gesunden geeignet.

**AK (Ab):** Antikörper treten stets erst stimuliert durch die Antigene auf. In der akuten Phase ist der Antikörpertiter niedrig oder nicht nachweisbar, da die Antikörper in den Immunkomplexen gebunden werden. Besser nachweisbar sind die Antikörper in der Rekonvaleszenzzeit. (Anmerkung: Es wird darauf hingewiesen, daß die früher von dritter Seite publizierten Daten sich immer auf den Immunfluoreszenztest (IF) beziehen, der erheblich unempfindlicher ist, so daß nur ab und zu Antikörper nachweisbar waren.

In den nachfolgenden Anwendungsbeispielen wird gezeigt, daß sich der Zustand der Infektion dauernd verändert (besonders in der Krankheitsphase) und daß für diesen Typus einer persistierenden Virusinfektion (mit niedriger Replikationsrate während der Aktivierungsphasen) diagnostisch eine Erfassung mehrerer Parameter optimal ist.

### VII. Anwendungsbeispiele der Erfindung

Es werden im folgenden drei Fallbeispiele aus dem Humanbereich und zwei Fallbeispiele aus dem Tierbereich dargestellt. Die Beispiele wurden aus einer Vielzahl ausgewählt, weil besonders lange Beobachtungszeiten bis zu 7 Monaten mit einer überdurchschnittlich großen Probenzahl (bis zu 23 von einem Individuum) untersucht werden konnten.

### Humanpatient 1: (Initialen: D.R.)

- Geschlecht: männlich
- Alter: 59 Jahre
- Klinische Diagnose: rekurrente enodogene Depression (rMDD=recurrent major depressive disorder), zusätzlich neurot. Anteile
- Untersuchung: in der akuten depressiven Episode
- Beobachtungszeit: 27 Wochen (ca. 7 Monate, Mai-November 1996)
- Anzahl Proben: 23, davon 13 in wöchentl. Abstand
- Summarisches Testergebnis:
   Antigen: 4/23
   CIC: 23/23
   Antikörper: 0/23
- Verlauf im Detail: Tabelle 1

### Humanpatient 2: (Initialen: G.R.)

- Geschlecht: weiblich
- Alter: 76 Jahre
- Klinische Diagnose: rMDD
- Untersuchung: in der akuten depressiven Phase
- Beobachtungszeit: 21 Wochen (ca. 5 Monate, Juni bis November 1996)
- Anzahl Proben: 18 (davon 14 in wöchentlichem Abstand)
- Summarisches Testergebnis:
   Antigen: 4/18
   CIC: 17/18
   Antikörper: 3/18
- Verlauf im Detail: Tabelle 2

### Humanpatient 3: (Initialen A.J.)

- Geschlecht: männlich
- Alter: 63 Jahre
- Klinische Diagnose: rMDD
- Untersuchung: in der akuten depressiven Episode
- Beobachtungszeit: 6 Wochen (August bis September 1996)
- Anzahl der Proben: 7, alle wöchentlich
- Summarisches Ergebnis:
   Antigen: 1/7
   CIC: 4/7 (2 Proben +/-)
   Antikörper: 1/7
- Verlauf im Detail: Tabelle 3

### Pferdepatient 1: (A.S., aus Nordrheinwestfalen)

- Geschlecht: männlich, Wallach, Hannoveraner
- Alter: 11 Jahre
- Klinische Diagnose: Borna'sche Krankheit, schwere Episode von Apathie, Somnolenz, Inappetenz, verändertem Freßverhalten; Spontanremission (Arch Virol (1993) Suppl 13, in press)
- Untersuchung: in der akuten Krankheitsphase und nach Gesundung Beginn: 3 Wochen nach Erkrankungsbeginn)
- Beobachtungszeit: 28 Wochen (7 Monate, Juni 1995 bis Januar 1996)
- Probenanzahl: 8 (6 komplette Untersuchungen)
- Summarisches Ergebnis:
   Antigen: 4/6
   CIC: v4/6
   Antikörper: 5/6
- Verlauf im Detail: Tabelle 4

### Pferdepatient 2: (F.D.) aus Bayern

- Geschlecht: männlich, Bayernwallach
- Alter: 15 Jahre
- Klinische Diagnose: Borna'sche Krankheit, schwere neurologische Verlaufsform, Euthanasie (August 1996); erste Episode in 1994, diese mit Spontanremission
- Beobachtungszeit: 14 Wochen (Mai bis August 1996)
- Probenanzahl: 3 Blutproben, 1 Liquorprobe
- Summarisches Ergebnis
   Blut:
   Antigen: 2/3
   CIC: 3/3
   Antikörper: 0/3
   Liquor:
   AG: Befund +/0
   CIC: 1/1
   Antikörper: 0/1
- Verlauf im Detail: Tabelle 5

Die Altersverteilung der Humanpatienten in den gewählten Fallbeispielen ist untypisch. Es gibt entsprechende Verläufe aus allen Altersgruppen. Die Verläufe sind typisch für Affekterkrankungen mit Rekurrenz (entweder rMDD oder bipolare Erkrankung), untersucht in der akuten Krankheitsphase.

Im symptomfreien Intervall bleiben niedrige Konzentrationen von CIC in der Regel erhalten, während Antigen in PBMCs und Antikörper nicht mehr nachweisbar sind. Die Dauer der Persistenz der CICs und die Titerhöhe hängt von der Stärke der vorangegangenen Aktivierungsphase der BDV-Infektion ab. Nach unseren bisherigen Untersuchungen bestehen die CICs mindestens 3 Monate fort (nach Krankheitsepisode). Für sehr lange symptomfreie Intervalle (z.B. mehrere Jahre) gibt es keine systematische Erhebungen. Aus Einzeluntersuchungen wissen wir, daß auch die CICs verschwinden können. Zum Beginn einer neuen depressiven Episode nach langem gesunden Intervall kann der Status für alle 3 Parameter negativ sein bei der Eingangsuntersuchung. Um sicher eine BCV-Infektion ausschließen zu können, genügt deshalb eine einzige Untersuchung nicht. Vielmehr ist mindestens eine zweite im ersten Drittel der Krankheitsepisode notwendig. Wenn eine Infektion vorliegt, findet man in der Regel dann zumindest CICs bei noch negativem AG bzw. AK-Befund.

Dies bedeutet in der Praxis, daß die BDV-Infektion bei Berücksichtigung eines Zeitintervalls durch die in der Erfindung dargestellten CICs und den neuen Tripeltest, der AG und AK mit berücksichtigt, mit einer hinreichenden Sicherheit erkannt werden kann.

Die Pferdepatienten-Fallbeispiele berücksichtigen erkrankte, infizierte Tiere. Gesunde symptomfreie Träger der Infektion sind häufig nur durch CICs detektierbar. Beispiele für Untersuchungen in gesunden Beständen sind unter X. angegeben.

### Falldarstellungen von Sektion VII. im Detail:

**Tabelle 1: Humanpatient 1 (D.R.)**

| Probe | Testwoche | AG | CIC | CIC | AK |
|---|---|---|---|---|---|
| | | | | Ext. 1:20 | |
| 1889 | 0 | (+) | +++ | 0,982 | 0 |
| 1960 | 3 | 0 | ++++ | 1,019 | 0 |
| 1995 | 4 | 0 | +++ | 0,858 | 0 |
| 2026 | 5 | 0 | + | 0,241 | 0 |
| 2054 | 6 | 0 | (+) | 0,129 | 0 |
| 2097 | 7 | 0 | ++ | 0,323 | 0 |
| 2111 | 8 | 0 | ++ | 0,503 | 0 |
| 2153 | 9 | 0 | ++ | 0,582 | 0 |
| 2174 | 10 | 0 | ++++ | 1,237 | 0 |
| 2204 | 11 | 0 | +++ | 0,911 | 0 |
| 2224 | 12 | 0 | ++ | 0,663 | 0 |
| 2271 | 13 | 0 | ++ | 0,366 | 0 |
| 2286 | 14 | + | +++ | 0,790 | 0 |
| 2341 | 15 | 0 | ++++ | 1,179 | 0 |
| 2374 | 16 | 0 | ++ | 0,429 | 0 |
| 2413 | 18 | + | ++ | 0,397 | 0 |
| 2471 | 20 | (+) | +++ | | 0 |
| 2502 | 21 | 0 | +++ | | 0 |
| 2528 | 22 | +/- | +++ | | 0 |
| 2575 | 23 | 0 | ++ | | 0 |
| 2627 | 24 | 0 | +++ | | 0 |
| 2655 | 25 | 0 | ++ | | 0 |
| 2723 | 27 | 0 | + | | 0 |

| | | | | | |
|---|---|---|---|---|---|
| Negativkontrolle bei <0,1 Extinktion | | | | | |

### Kommentar:

Humanpatient 1 stellt insofern einen Sonderfall dar, als er zu den wenigen Patienten zählt, bei denen in der akuten Krankheitsphase bei wöchentlichen Kontrollen faßt ausschließlich exorbitant hohe CIC-Konzentrationen, aber keine freien Antikörper und nur selten AG in PBMCs nachweisbar waren (siehe auch Einführungsteil). Wir nehmen an, daß das in den PBMCs gebildete BDV-AG sofort ins Plasma übertritt und von den vorhandenen Antikörpern in CICs gebunden wird. Solche Patienten wären mit herkömmlichen Testmethoden, ohne Berücksichtigung der CICs praktisch nicht erfaßbar.

Wir haben die ersten 11 Testwochen nicht nur in dem unter C beschriebenen klassischen Testversion untersucht, sondern durch Einsatz nur jeweils eines monoklonalen "Catch"-Antikörpers (Testschritt A2) die Menge der jeweils mit p40 oder mit p24 gebildeten CICs bestimmt. In der Ergänzungstabelle wird deutlich, daß die Mengen von p40-CICs und p24-CICs deutlich variieren und ein zeitliches Konzentrationsprofil erkennbar wird. Diese Daten werden auch als zusätzlicher Beweis für die sehr hohe Spezifität des CIC-Nachweises präsentiert. Je nach Vorbeschichtung in A2 (Wahl des "Catch"-AK) können also CICs auch für einzelne Virusproteine bestimmt werden.

### Ergänzungstabelle zu Humanpatient 1:

| Probe | Testwoche | p40-CIC | p40-CIC | p24-CIC | p24-CIC |
|---|---|---|---|---|---|
| | | | Ext. 1:20 | | Ext. 1:20 |
| 1889 | 0 | ++++ | 1,288 | ++ | 0,436 |
| 1960 | 3 | ++ | 0,496 | +++ | 0,807 |
| 1995 | 4 | +++ | 0,733 | +++ | 0,921 |
| 2026 | 5 | ++ | 0,318 | ++ | 0,399 |
| 2111 | 8 | +++ | 0,809 | ++ | 0,369 |
| 2153 | 9 | ++++ | 1,336 | +++ | 0,847 |
| 2174 | 10 | ++++ | 1,407 | ++++ | 1,032 |
| 2204 | 11 | +++ | 0,830 | +++ | 0,820 |

| | | | | | |
|---|---|---|---|---|---|
| Negativkontrolle bei 0,05 Extinktion | | | | | |

**Tabelle 2: Humanpatient 2 (G.R.)**

| Probe | Testwoche | AG | CIC | CIC | AK |
|---|---|---|---|---|---|
| | | | | Ext. 1:20 | |
| 2025 | 0 | 0 | (+) | | 0 |
| 2051 | 1 | 0 | + | | 0 |
| 2108 | 2 | 0 | + | | 0 |
| 2151 | 3 | 0 | + | | 0 |
| 2173 | 4 | + | ++ | | 0 |
| 2203 | 5 | 0 | + | | 0 |
| 2222 | 6 | (+) | + | | 0 |
| 2269 | 7 | 0 | + | | 0 |
| 2287 | 8 | 0 | +++ | | +(1:10) |
| 2340 | 9 | 0 | ++ | | 0 |
| 2369 | 10 | 0 | +++ | | 0 |
| 2419 | 12 | + | + | | +(1:10) |
| 2454 | 13 | 0 | + | | 0 |
| 2477 | 14 | 0 | + | | 0 |
| 2503 | 15 | 0 | +++ | | 0 |
| 2530 | 16 | (+) | ++ | | 0 |
| 2647 | 19 | 0 | ++ | | +(1:10) |
| 2731 | 21 | 0 | 0 | | 0 |

### Kommentar:

Der hier dargestellte Verlauf der Infektionsparameter im Krankheitsverlauf trifft für viele infizierte Patienten in ähnlicher Form zu. Der Akutmarker "AG in PBMCs" ist in Form von Peaks nachweisbar, jedoch nicht als mehrere Wochen überspannendes Plateau. Die CIC-Messung zeigt, daß Antigen aber dauerhaft in Schüben gebildet wird und nach Ausschleusung aus den Zellen von Antikörpern komplexiert wird. Freie Antikörper sind daher auch nur sporadisch im gesamten Intervall nachweisbar.

**Tabelle 3: Humanpatient (A. J.)**

| Probe | Testwoche | AG | CIC | CIC | AK |
|---|---|---|---|---|---|
| | | | | Ext. 1:20 | |
| 2270 | 0 | 0 | +/- | 0,149 (1:40) | 0 |
| 2285 | 1 | + | + | 0,231 | 0 |
| 2339 | 2 | 0 | + | 0,231 | |
| 2370 | 3 | 0 | 0 | 0,056 | + (1:10) |
| 2400 | 4 | 0 | ++ | 0,544 | 0 |
| 2418 | 5 | 0 | + | | 0 |
| 2455 | 6 | 0 | +/- | | 0 |

### Kommentar:

In den ersten 4 Testwochen konnten AG in PBMCs, CICs und Antikörper nachgewiesen werden. Der zuverlässigste Parameter war wieder in Form der CICs sichtbar. Dies Beispiel zeigt, daß auch im Fall einer verhältnismäßig niedrigen und kurzen Antigenproduktion CICs nachweisbar sind, allerdings dann nur in niedrigen Konzentrationen.

**Tabelle 4: Pferdepatient 1 (A.S.)**

| Probe | Testwoche | AG | CIC | CIC | AK |
|---|---|---|---|---|---|
| | | | | Ext. 1:20 | |
| 253 | 0 | + | kein Test | | + (1:20) |
| 316 | 3 | + | kein Test | | + (1:10) |
| 432 | 8* | ++++ | + | | + (1:20) |
| 534 | 11** | + | ++ | | + (1:20) |
| 908 | 15 | + | 0 | | 0 |
| 990 | 19 | +/- | 0 | | 1 (1:10) |
| 1126 | 23 | 0 | (+) | | ++ (1:40) |
| 1216 | 28 | 0 | + | | + (1:10) |

| | | | | | |
|---|---|---|---|---|---|
| * = 11. Krankheitswoche: sehr krank; ** ca. 13 Krankheitswoche: deutliche Besserung; leichter Rückfall in 18. Kranhkeitswoche (15. Testwoche); ab 20. Krankheitswoche stete Besserung bis zur vollständigen Gesundung. | | | | | |

### Kommentar:

Dieses Pferd hat eine ungewöhnlich schwere und lange Erkrankungsphase durchgemacht, die dennoch zur völligen Ausheilung kam. Die Häufigkeit der positiven Antikörpertests ist allerdings eher untypisch und vermutlich durch die sehr starke Antigenproduktion in PBMCs (auch selten so hoch) bedingt. Bei kürzeren Krankheitsphasen sind die Antikörper in der Regel ebenso sporadisch und in niedrigen Titern nachweisbar wie bei den depressiven Humanpatienten.

**Tabelle 5: Pferdepatient 2 (F.D.)**

| Probe | Testwoche | AG | CIC | CIC | AK |
|---|---|---|---|---|---|
| | | | | Ext. 1:20 | |
| 1871 | 0 | 0 | ++++ | | 0 |
| 1972 | 4 | + | ++ | | 0 |
| 2279 | 14 | + | ++++ | | 0 |
| Liquor | 14 | +/- | + | | 0 |

### Kommentar:

Dieses Pferd ist an der Borna'schen Krankheit gestorben bzw. mußte eingeschläfert werden. In der akuten Krankheitsphase waren dennoch keine freien Antikörper im Plasma nachweisbar. Diese Infektion wäre trotz schwerer Krankheit nur mit serologischen Methoden nicht verifizierbar gewesen. Bei diesem Pferd sind exorbitante CIC-Titer gefunden worden und AG in PBMCS. Dies weist auf eine starke Aktivierungsphase der Virusinfektion hin (auch der Liquor war positiv), die letzlich außer Kontrolle geraten sein muß.

Exorbitant hohe CIC-Titer sind, wie bei Humanpatienten, auch bei BDV-infizierten Pferden eher selten.

### Auswertungsmöglichkeiten für AG und CIC-Tests:

In der Regel ist eine semiquantitative Auswertung, die die relative Konzentration der Parameter wiedergibt, einer Titerangabe vorzuziehen. Jeder Parameter wird in einer 8-stufigen Verdünnungsreihe pro Probe gemessen. Die parallel zur Verdünnung abnehmende Extinktion gibt eine zusätzliche Sicherheit in der Titerbeurteilung. Sie kann, aber muß nicht für eine Endpunkttiterbestimmung herangezogen werden.

### Auswertung AG-Tests:

Für die Bestimmung wird die Zellsuspension oder Blutplasmaprobe ab 1:2 verdünnt.
Die negative Kontrolle ist in der Regel 0,05 bis <0,1 Extinktion.

| | | |
|---|---|---|
| +/- | grenzwertig | 0,1 - 0,12 bei 1:20 und klarer Verdünnungsreihe |
| (+) | schwach positiv | >0,12 - 0,15 bei 1:20 und klarer Verdünungsreihe |
| + | positiv | >0,15 - 0,25 bie 1:20 |
| ++ | mittelstark positiv | >0,25 - 0,45 bei 1:20 |
| +++ | stark positiv | >0,45 bis 1,0 bei 1:20 |
| ++++ | sehr stark positiv | >1,0 und höher bei 1:20 |

Die meisten CIC-Werte liegen im mittleren Extinktionsbereich. Für eine positive Beurteilung muß immer eine verdünnungsabhängige Extinktionsabnahme erkennbar sein. Grenzwertige Teste sind sehr selten, weil keine Hintergrundfärbung auftritt. Die angegebenen Extinktionsbereiche sind als Richtwerte zu verstehen.

### Auswertung AK-Teste (EIA, Test D)

Für die Bestimmung wird das Plasma ab 1:50 verdünnt.
Die negative Kontrolle ist in der Regel etwas höher als bei CIC und AG-Test (leichte Hintergrundfärbung): 0,1 bis <0,2 Extinktion.

| | | |
|---|---|---|
| +/- | grenzwertig | 0,2 bis 0,3 bei 1:50 und 1:100, dann Abfall |
| (+) | schwach positiv | >0,3 - 0,4 bei 1:50, klare Verdünnungsreihe |
| + | positiv | >0,4 - 0,6 bei 1:50 |
| ++ | mittelstark positiv | >0,6 - 0,8 bei 1:50 |
| +++ | stark postiv | >0,8 und darüber bei 1:50 |

Für eine postive Beurteilung muß immer eine verdünnungsabhängige Extinktionsabnahme erkennbar sein. Die Basisextinktion der Negativkontrolle sollte nicht >0,2 sein.

Für die Auswertung der AK-Teste ist eine Titerangabe der semiquantitativen Auswertung vorzuziehen, wenn Vergleiche mit Testen anderer Labors gezogen werden sollen. Beispiele für die AK-Teste sind unter IX. angegeben.

### VIII. Bewertung des aktuellen Zustandes der BDV-Infektion mit der Testkombination

Der AG-Nachweis in PBMCs (ebenso wie im Blutplasma) ist ein Parameter für einen akuten Aktivierungsprozess. Der CIC-Parameter beurteilt das zurückliegende Intervall bei Verlaufsuntersuchungen. Wenn CIC-Werte ansteigen im Intervall, bedeutet das, daß Virusantigen gebildet worden ist und zum Testzeitpunkt bereits in komplexierter Form vorliegt. Ob freie Antikörper nachweisbar sind, wird durch die Antikörperproduktion im Verhältnis zur Antigenproduktion bestimmt. Wenn die Antikörperproduktion gerade erst infolge eines Aktivierungsschubs neu begonnen hat, werden kaum freie Antikörper, dafür aber CICs nachweisbar sein. Zu einem späteren Zeitpunkt, bei nachlassender Antigenproduktion, steigt die Chance zum Nachweis freier Antikörper (mit oder ohne gleichzeitigen CIC-Nachweis).

Eine diese Verhältnisse berücksichtigende Interpretation des Tripeitestts ist sehr hilfreich für die Beurteilung klinischer Verläufe.

Folgende Testkonstellationen sind nach dem heutigen Stand möglich:
1. AG negativ CIC negativ AK positiv Infektion liegt vor, ist aber nicht aktiviert, weder akut noch in den letzten Wochen.
2. AG positiv CIC negativ AK negativ Infektion ist gerade ganz akut aktiviert zum Untersuchungszeitpunkt, nicht vorher.
3. AG positiv CIC positiv AK negativ oder positiv Infektion liegt vor, Aktivierungsbeginn liegt schon einige Wochen zurück, Aktivierung ist aber zum Untersuchungszeitpunkt noch akut.
4. AG negativ CIC positiv AK negativ oder positiv Infektion liegt vor, Aktivierungsbeginn liegt schon einige Wochen, eventuell Monate (je nach Untersuchungsintervall) zurück. Aktivierung direkt zum Untersuchungszeitpunkt nicht mehr vorhanden.
   Bei Erstuntersuchung kann nur die Aussage: "Infektion liegt vor" getroffen werden.
5. AG negativ CIC negativ AK negativ kein Hinweis auf eine BDV-Infektion. Bei zwei komplett negativen Testen in der akuten Krankheitsphase kann eine Infektion ausgeschlossen werden.

Mindestens zwei komplett negative Teste (Abstand 4 - 6 Wochen) sind auch als Therapiekontrolle vor Beendigung einer antiviralen Behandlung (Lancet (1997) 349: 178-179) zu fordern.

Bisherige Untersuchungen haben gezeigt, daß CICs ein sehr sensitiver Marker sind zur Beurteilung des antiviralen Therapie-Erfolgs. Sie sind als letzter Parameter nach bereits erfolgter klinischer Besserung oft noch nachweisbar, verschwinden aber bei Fortsetzung der Therapie später.

### IX. Beispiele für den AK-Test, parallel zu CIC und AG

Anders als bei den Falldarstellungen, für die Entwicklung der Virusparameter während einer Krankheitsphase exemplarisch gezeigt wurde, sollen hier eine stark positive Humanprobe (1437) und eine schwach positive (für AG vollständig negative) Probe (890) in kompletten Verdünnungsreihen dargestellt werden. Beide Proben stammen aus USA und wurden nach einem 4 Tage dauernden Transport in 4/94 getestet in denselben Testansätzen.

| Testergebnis: | | | Testergebnis: | |
|---|---|---|---|---|
| AG (p40 + p24): | +++ | (1:16 - 1: 32) | 0 | (<1:2) |
| AG (p40): | +++ | (1:8) | 0 | (<1:2) |
| AG (p24): | ++ | (1:8) | 0 | (<1:2) |
| | | | | |
| CIC (p40 + p24): | ++ | (1:640) | +/0 | (1:20) |
| CIC (p40): | +++ | (1:1280) | + | (1:80) |
| CIC (p24): | ++ | (1:640) | + | (1:80 - 1:160) |
| | | | | |
| AK (p40 + p24): | + | (1:400) | +/0 | (1:50) |
| AK (p40) | ++ | (1:1600) | + | (1:100 - 1:200) |
| AK (p24) | + | (1:400 - 800) | (+) | (1:50 - 1:100) |

### Verdünnungsreihen Probe 1437:

### ANTIGEN - 1437

| Verdünnung | AG p40 + p24 Ext. | AG p40 Ext. | AG p24 Ext. |
|---|---|---|---|
| | | | |
| 1:2 | 0,924 | 1,135 | 0,853 |
| 1:4 | 0,337 | 0,424 | 0,272 |
| 1:8 | 0,162 | 0,170 | 0,138 |
| 1:16 | 0,115 | 0,076 | 0,078 |
| 1:32 | 0,127 | 0,047 | 0,055 |
| 1:64 | 0,095 | 0,052 | 0,052 |
| 1:128 | 0,100 | 0,054 | 0,054 |
| 1:256 | 0,098 | 0,060 | 0,059 |

| | | | |
|---|---|---|---|
| Negativkontrolle: 0,04 - 0,05 | | | |

### CIC - 1437

| Verdünnung | CIC p40 + p24 | CIC p40 | CIC p24 |
|---|---|---|---|
| | Ext. | Ext. | Ext. |
| | | | |
| 1:20 | 0,515 | 0,777 | 0,654 |
| 1:40 | 0,267 | 0,736 | 0,608 |
| 1:80 | 0,258 | 0,498 | 0,546 |
| 1:160 | 0,202 | 0,476 | 0,430 |
| 1:320 | 0,167 | 0,406 | 0,344 |
| 1:640 | 0,107 | 0,319 | 0,271 |
| 1:1280 | 0,077 | 0,257 | 0,201 |
| 1:2560 | 0,063 | 0,248 | 0,218 |

Negativkontrolle für CIC p40 + p24 (= Standardtest) 0,05; für die Einzeltests 0,1 bis 0,15, untypisch, etwas erhöhter "Background".

### ANTIKÖRPER - 1437

| Verdünnung | AK p40 + p24 | AK p40 | AK p34 |
|---|---|---|---|
| | Ext. | Ext. | Ext. |
| | | | |
| 1:50 | 0,249 | 0,767 | 0,513 |
| 1:100 | 0,172 | 0,606 | 0,375 |
| 1:200 | 0,134 | 0,513 | 0,364 |
| 1:400 | 0,120 | 0,395 | 0,296 |
| 1:800 | 0,108 | 0,310 | 0,217 |
| 1:1600 | 0,076 | 0,225 | 0,172 |
| 1:3200 | 0,045 | 0,165 | 0,112 |
| 1:6400 | 0,041 | 0,154 | 0,113 |

| | | | |
|---|---|---|---|
| Negativkontrolle 0,08 - 0,100 (Wie beschrieben, kann der "Background"-Level höher sein, bis 0,200) | | | |

### Verdünnungsreihen Probe 890

### ANTIGEN - 890

| Verdünnung | CIC p40 + p24 | CIC p40 | CIC p24 |
|---|---|---|---|
| | Ext. | Ext. | Ext. |
| | | | |
| 1:20 | 0,177 | 0,267 | 0,293 |
| 1:40 | 0,076 | 0,267 | 0,237 |
| 1:80 | 0,079 | 0,227 | 0,156 |
| 1:160 | 0,072 | 0,161 | 0,130 |
| 1:320 | 0,090 | 0,126 | 0,101 |
| 1:640 | 0,074 | 0,122 | 0,100 |
| 1:1280 | 0,069 | 0,101 | 0,092 |
| 1:2560 | 0,126 | 0,105 | 0,098 |

| | | | |
|---|---|---|---|
| Kontrollen wie bei 1437 | | | |

### ANTIKÖRPER - 890

| Verdünnung | AK p40 + p24 | AK p40 | AK p24 |
|---|---|---|---|
| | Ext. | Ext. | Ext. |
| | | | |
| 1:50 | 0,158 | 0,320 | 0,207 |
| 1:100 | 0,083 | 0,211 | 0,118 |
| 1:200 | 0,077 | 0,164 | 0,107 |
| 1:400 | 0,075 | 0,092 | 0,082 |
| 1:800 | 0,087 | 0,071 | 0,064 |
| 1:1600 | 0,064 | 0,065 | 0,055 |
| 1:3200 | 0,045 | 0,056 | 0,039 |
| 1:6400 | 0,028 | 0,069 | 0,059 |

| | | | |
|---|---|---|---|
| Kontrollen wie bei 1437 | | | |

### Kommentar:

Die Probe 1437 zeigte in den PBMCs ungewöhnlich hohe Antigenexpression. Im Durchschnitt liegen die Werte deutlich niedriger. Auf Probe 1437 trifft die Konstellation 3 (siehe VIII, Bewertung) zu, d.h. AG positiv, CIC positiv und AK positiv. Die Aktivierungsphase der Infektion ist akut, besteht aber schon einige Zeit. Bemerkenswert ist noch, daß der Standard-Immunfluoreszenztest nur einen gerade noch nachweisbaren Titer von 1:10 ergeben hat, während der AK-Test des Tripel-Testsets auftrund der höheren Sensivität ein eindeutig positives AK-Ergebnis erbrachte.

Die Probe 890 war im Antigentest in den PBMCs vollständig negativ. Der Standard-Antikörpertest mit Immunfluoreszenz war ebenfalls negativ. Mit diesen beiden Parametern wäre die latente Infektion nicht erkannt worden. Der CIC-Test für beide Antigene und der entsprechende AK-Test (Tripelset) waren grenzwertig, d.h. es bestand Infektionsverdacht. In solchen Fällen bietet die Anwendung der Teste für die Einzelantigene, wie oben demonstriert, eine Chance zur Abklärung. Ansonsten muß in solchen Fällen eine weitere Folgeprobe untersucht werden, um zu einem eindeutigen Ergebnis zu gelangen. Auf die Probe 890 trifft die Konstellation 4 (bzw. 5) in VIII. (Bewertung) zu.

### X. Infektionsnachweis in symptomfreien Pferdebeständen

Von August bis Oktober 1995 wurden 222 gesunde Vollblutzuchtpferde aus 33 Gestüten auf BDV untesucht. Dabei wurden AG-Test und CIC-Test aus der Tripelkombination eingesetzt. Die Antikörper wurden mit dem Standardimmunfluoreszenztest ermittelt. Die durchschnittlich pro Gestüt untersuchte Anzahl von Pferden war a6,7 +- 5,1.

Eine 100%ige Durchseuchung fanden wir bei 14 von 33 Gestüten (= 42,4%). AG in PBMCs als Zeichen für eine aktivierte Infektion ergab sich nur bei 38 von 221 Pferden (= 17,2%). AK (IF-Test) wurden bei 68 von 222 (= 30,6%) nachgewiesen. Beide Prozentsätze entsprechen anderen Untersuchungen in gesunden Beständen. Die tatsächliche Durchseuchung wird aber erst durch den CIC-Test sichtbar. Positive CICs fanden wir bei 156 von 219 Pferden (= 71,2%). Bei einem guten Viertel (26,2%) der Pferde waren CICs der einzige Infektionsmarker (58 von 221 Tieren), d.h. jedes vierte gesunde Tier war latent infiziert, konnte aber nur über den CIC-Parameter detektiert werden.

Nachfolgend werden aus dieser Gruppe am Beispiel von 6 Tieren aus 4 Gestüten die Untersuchungsergebnisse mit vollständiger Titration der CIC-Werte zusammengestellt.

### Beispiel 1:

| | | | | |
|---|---|---|---|---|
| Labor-Nr. 475 | Gestüt B | Stute C | Alter 15 Jahre | immer gesund |
| Labor-Nr. 529 | Gestüt W | Hengst HH | Alter 25 Jahre | immer gesund |

untersucht August und September 1995:

| Nr. 475 | | | | | Nr. 529 | | | |
|---|---|---|---|---|---|---|---|---|
| BDV-AG | | neg. | BDV-AK | neg. | BDV-AG | neg. | BDV-AK | neg. |
| CIC | | ++++ | | | CIC | + | | |
| 1:20 | | 1,020 | | | 1:20 | 0,210 | | |
| 1:40 | | 0,753 | | | 1:40 | 0,148 | | |
| 1:80 | | 0,641 | | | 1:80 | 0,163 | | |
| 1:160 | | 0,450 | | | 1:60 | 0,151 | | |
| 1:320 | .... | 0,317 | | | 1:320 | 0,133 | | |
| 1:640 | | 0,243 | | | 1:640 | 0,121 | | |
| 1:1280 | | 0,243 | | | 1:1280 | 0,112 | | |
| 1:2560 | | 0,212 | | | 1:2560 | 0,130 | | |

### Beispiel 2:

| | | | | |
|---|---|---|---|---|
| a) Labor-Nr. 495 | Gestüt G | Stute Z | Alter 18 Jahre | immer gesund |
| b) Labor-Nr. 496 | Gestüt G | Sute W | Alter 21 Jahre | immer gesund |

untersucht August 1995:

| Nr. 495 | | | | Nr. 496 | | | |
|---|---|---|---|---|---|---|---|
| BDV-AG | neg. | BDV-AK | neg. | BDV-AG | neg. | BDV-AK | neg. |
| CIC | ++ | | | CIC | 0 | | |
| 1:20 | 0,445 | | | 1:20 | 0,104 | | |
| 1:40 | 0,445 | | | 1:40 | 0,126 | | |
| 1:80 | 0,301 | | | 1:80 | 0,124 | | |
| 1:160 | 0,202 | | | 1:160 | 0,095 | | |
| 1:320 | 0,178 | | | 1:320 | 0,083 | | |
| 1:640 | 0,154 | | | 1:640 | 0,110 | | |
| 1:1280 | 0,138 | | | 1:640 | 0,124 | | |
| 1:2560 | 0,148 | | | 1:640 | 0,126 | | |

### Beispiel 3:

| | | | | |
|---|---|---|---|---|
| a) Labor-Nr. 521 | Gestüt R | Hengst W | Alter 23 Jahre | immer gesund |
| b) Labor-Nr. 522 | Gestüt R | Stute MD | Alter 9 Jahre | immer gesund |

untersucht September 1995:

| Nr. 521 | | | | Nr. 522 | | | |
|---|---|---|---|---|---|---|---|
| BDV-AG | neg. | BDV-AK | neg. | BDV-AG | neg. | BDV-AK | neg. |
| CIC | +++ | | | CIC | ++++ | | |
| 1:20 | 1,072 | | | 1:20 | 1,362 | | |
| 1:40 | 0,337 | | | 1:40 | 1,154 | | |
| 1:80 | 0,255 | | | 1:80 | 0,887 | | |
| 1:160 | 0,188 | | | 1:160 | 0,660 | | |
| 1:320 | 0,198 | | | 1:320 | 0,431 | | |
| 1:640 | 0,168 | | | 1:640 | 0,329 | | |
| 1:1280 | 0,165 | | | 1:1280 | 0,263 | | |
| 1:2560 | 0,165 | | | 1:2560 | 0,241 | | |

### Kommentar:

Am Beispiel 1 werden hoch-positive und niedrig-positive CIC-Ergebnisse gegenübergestellt. Trotz des hohen Alters von Hengst 1b (Nr. 529) von 25 Jahren müssen die CIC-Werte nicht zwangsläufig hoch sein.

Das Beispiel 2 zeigt zwei Stuten aus demselben Gestüt in vergleichbarem Alter. Stute 2a (495) ist infiziert und hatte mittlere CIC-Werte. Stute 2b 8496) war dagegen CIC-negativ und nicht infiziert.

Am Beispiel 3 werden zwei hoch-positive Tiere aus demselben Gestüt aus unterschiedlichen Altersgruppen gezeigt (23 und 9 Jahre). Das jüngere Tier hatte exorbitant hohe CIC-Werte.

Für alle Tiere gilt gleichermaßen, daß es hochwertige Zuchtpferde sind, die weder früher noch zum Untersuchungszeitpunkt krank waren.

Die Erfindung ermöglicht erstmalig eine spezies-übergreifende, (nahezu) lückenlose Erfassung der verschiedenen Phasen einer BDV-Infektion durch Kombination des neu entdeckten Testparameters "zirkulierende Immunkomplexe" (CICs) im Blutplasma, mit bereits bekannten Parametern, nämlich Antigen (AG) in weißen Blutzellen und Antikörpern (AK) im Plasma. Alle drei Parameter können mit einer einzigen Blutprobe bestimmt werden. Der in der Erfindung erstmalig beschriebene Nachweis von BDV-spezifischen CICs als einzigen längerfristig persistierenden Infektionsmarkern ermöglicht sowoh die Erfassung gesunder Träger (latenter Infektionen) als auch die Beurteilung von Infektionsverlauf und Therapieerfolg bei erkrankten Individuen. Dabei werden an einer zu untersuchenden Körperflüssigkeitsprobe in der Körperflüssigkeit frei zirkulierende Immunkomplexe aus BDV-Antigenen und hieran angelagerten spezifischen Antikörpern durch einen geeigneten immunologischen Test nachgewiesen.

Im folgenden werden noch einige weitere Beispiele anhand von Figuren beschrieben

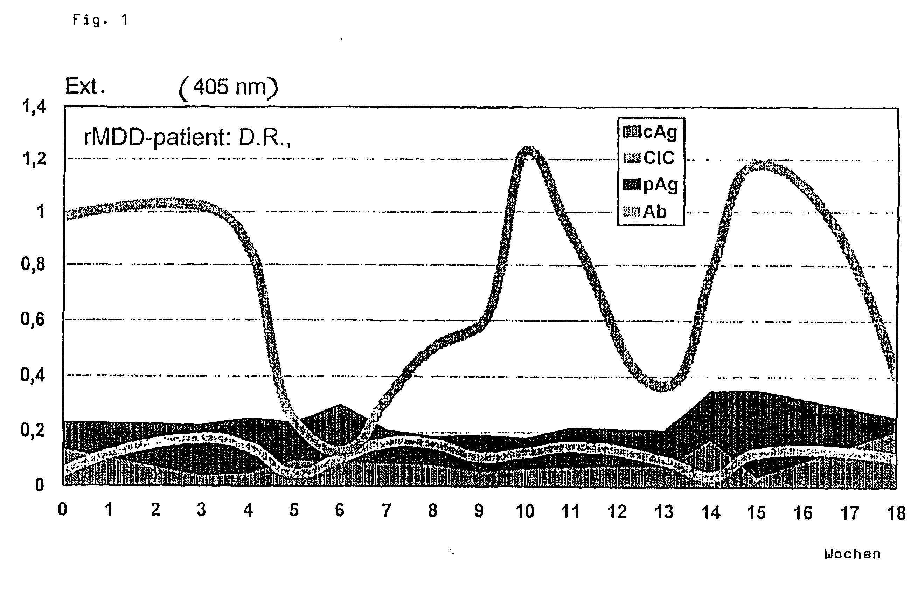

In den Figuren 1, 2, 4 und 5 wird schematisch gezeigt, wie sich der Zustand der Infektion (besonders in der Krankheitsphase) dauernd verändert und daß für diesen Tyjpus einer persistierenden Virusinfektion (mit niedriger Replikationsrate während der Aktivierungsphasen) diagnostisch eine Erfassung mehrerer Parameter optimal ist. Aufgetragen ist jeweils die Extinktion bei 405 nm über "Wochen" (Untersuchungsverlauf). Die Abbildungen zeigen den Verlauf für
CIC - zirkulierende Immunkomplexe
cAg - zelluläres Antigen
pAg - Antigen im Plasma
Ab - Antikörper (EIA)

Figur 1 zeigt die Daten des humanpatienten 1 (Initialen D.R.), eines rMDD-Patienten im Alter von 59 Jahren während stationärer klinischer Betreuung (Behandlung mit Antigendepressiva, nicht antiviral). Der Untersuchungszeitraum betrug ca. 3 Monate (18 Wochen). Testsystem: EIA; Verdünnung: cAG 1:2, CIC 1:20, pAG 1:2, Ab 1:100; Ausschlußgrenze 0,1 Probenursprung: für cAG: für cAG ultrabeschallte PBMCs, sonst entsprechende Plasmaproben.

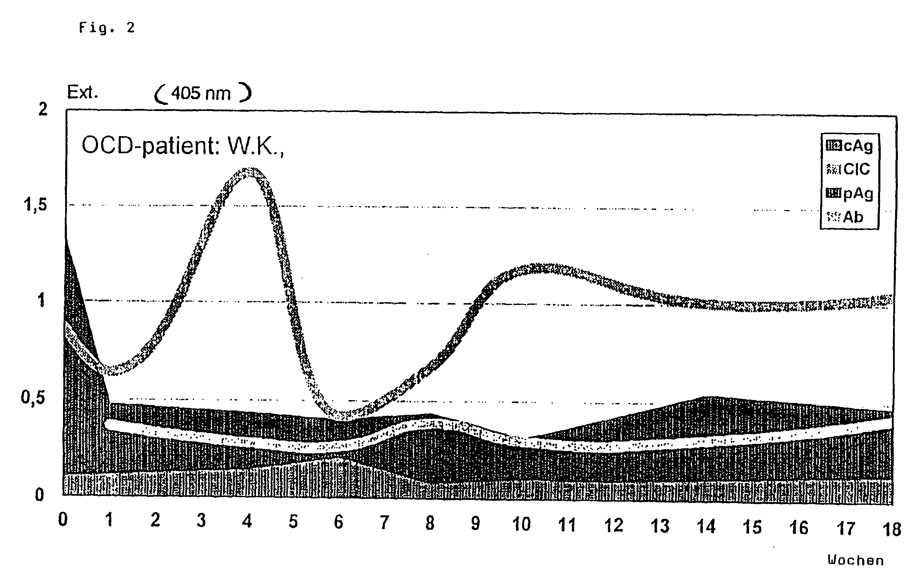

Figur 2 zeigt ein Figur 1 entsprechendes Diagramm für einen 30-jährigen OCD-Patienten (OCD = obsessive compulsive disorder) während stationärer klinischer Behandlung). Die BDV-Infektion spielt bei diesen Patienten ebenso wie bei den depressiven Patienten eine nicht unerhebliche Rolle. Bei beiden Patientengruppen - depressiven wie OCD-Patienten - können serotoningesteuerte Vorgänge gestört sein. Dies könnte damit zusammenhängen, da das BDV-Virus funtionell bestimmte Positionen im Gehirn und an den Nervenknoten angreift. Figuren 1 und 2 zeigen untereinander ähnliche Parameterentwicklungen.

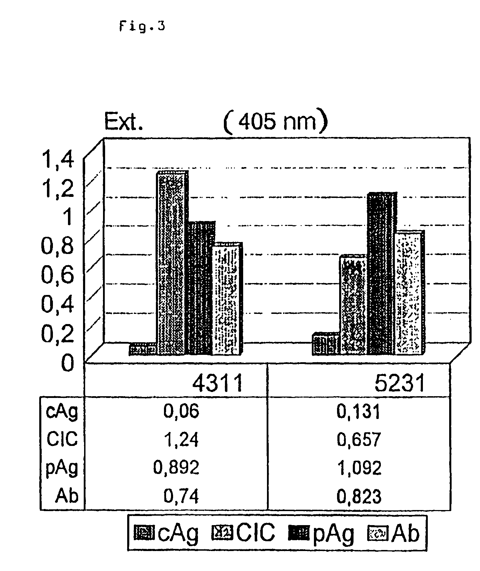

Figur 3 zeigt 2 Beispiele aus dem Tierbereich, und zwar zwei Bornakranke Pferde. Hier wurde jeweils nru eine Probe untersucht. Gut zu erkennen ist das Verhältnis von CIC zu pAG (freies Plasmaantigen) und Ab (= freie Plasmaantikörper (AK), gemessen mit EIA). Probe 4311 wurde von einem Pferd aus Norddeutschland (Initiale G.) gewonnen, das im März 1997 aufgrund der (fraglichen) klinischen Diagnose "Bornakrankheit) untersucht worden war. Der IF-Antikörpertiter betrug 1:40. Probe 5231 wurde von einem Pferd aus Süddeutschland (Initiale D.) gewonnen, das im Mai 1997 aufgrund der klinischen Diagnose Borna'sche Krankheit untersucht worden war. Der IF-Antikörpertest war negativ. Das Pferd war zwei Monate vorher, im März 1997, erkrankt und zeigte Apathie und Somnolenz. Im Mai noch verbliebene Symptome waren Kopfschütteln und steifer Gang (Meßpara-meter zu EIA: Verdünnung cAG 1:2, CIC 1:20, pAG 1:2, Ab 1:100, Ausschlußgrenze = 0,1, Probengrundlage: cAg: ultrabeschallte PBMCs, sonst entsprechende Plasmaproben).

Die den Abbildungen zugrundeliegenden Daten werden im folgenden in Tabellenform wiedergegeben:

**Tabelle 1 (zu Figur 1):**

| Ext.(405nm) für | cAG | CIC | pAG | Ab |
|---|---|---|---|---|
| 0 Wo. | 0,14 | 0,982 | 0,237 | 0,051 |
| 3 Wo. | 0,041 | 1,019 | 0,225 | 0,177 |
| 4 Wo. | 0,051 | 0,858 | 0,251 | 0,146 |
| 5 Wo. | 0,089 | 0,241 | 0,237 | 0,048 |
| 6 Wo. | 0,096 | 0,120 | 0,299 | 0,104 |
| 7 Wo. | 0,088 | 0,323 | 0,209 | 0,163 |
| 8 Wo. | 0,081 | 0,503 | 0,185 | 0,158 |
| 9 Wo. | 0,056 | 0,582 | 0,192 | 0,11 |
| 10 Wo. | 0,068 | 1,237 | 0,18 | 0,125 |
| 11 Wo. | 0,075 | 0,91 | 0,217 | 0,148 |
| 13 Wo. | 0,079 | 0,366 | 0,208 | 0,097 |
| 14 Wo. | 0,178 | 0,79 | 0,35 | 0,041 |
| 15 Wo. | 0,041 | 1,179 | 0,353 | 0,126 |
| 18 Wo. | 0,028 | 0,397 | 0,254 | 0,108 |

**Tabelle 2 (zu Figur 2):**

| Ext.(405nm) für: | cAG | CIC | pAG | Ab |
|---|---|---|---|---|
| 0 Wo. | 0,108 | 0,869 | 1,335 | |
| 1 Wo. | 0,117 | 0,639 | 0,478 | 0,367 |
| 4 Wo. | 0,146 | 1,679 | 0,438 | 0,28 |
| 6 Wo. | 0,208 | 0,421 | 0,401 | 0,263 |
| 8 Wo. | 0,08 | 0,675 | 0,439 | 0,386 |
| 10 Wo. | 0,105 | 1,174 | 0,307 | 0,296 |
| 12 Wo. | 0,096 | | | |
| 14 Wo. | | 1.009 | 0,542 | 0,307 |
| 18 Wo. | 0,134 | 1,048 | 0,474 | 0,417 |

**Tabelle 3 (zu Figur 3):**

| | Probe 4311 (Ext.) | Probe 5231 (Ext.) |
|---|---|---|
| cAG | 0,06 | 0,131 |
| CIC | 1,24 | 0,657 |
| pAG | 0,892 | 1,092 |
| Ab | 0,74 | 0,823 |

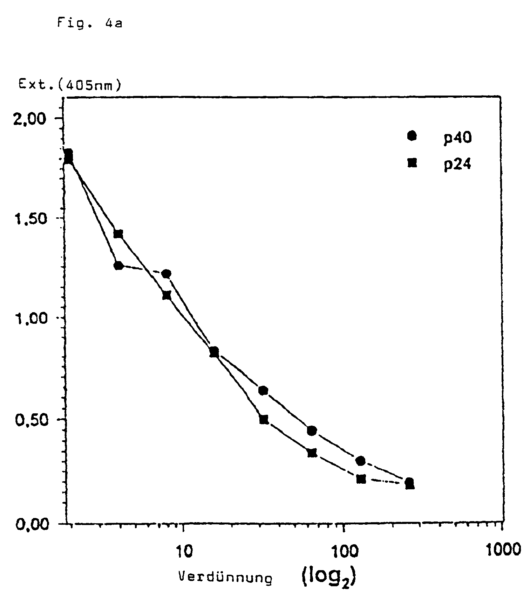

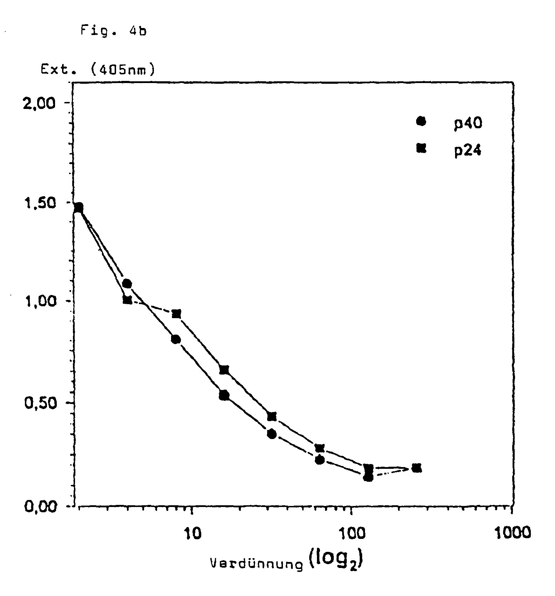

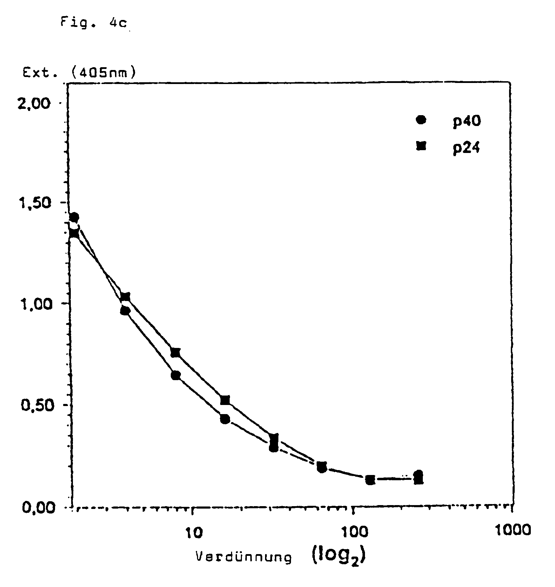

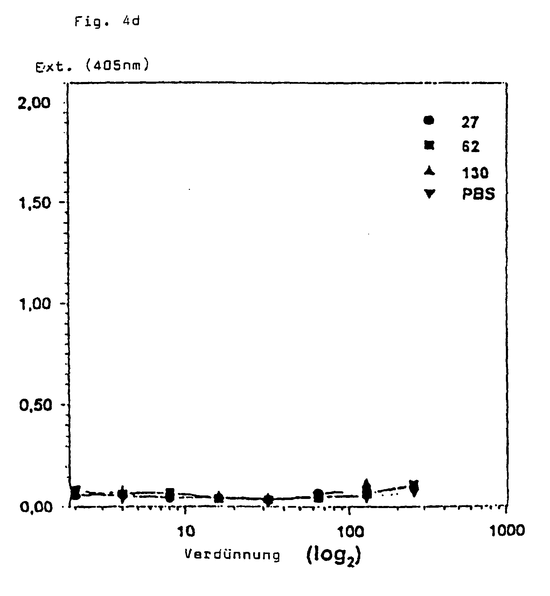

Figur 4 a) bis d) schließlich zeigt verschiedene Ergebnisse an CSF-Probenin verschiedener Verdünnung. Die Abbildungen a) bis c) zeigen hohe positive Antigenmarker bei Affektstörungen und d) negative Testergebnisse bei Schizophrenen.

Die hohen Werte sprechen für eine vorübergehend starke Virusvermehrung im Gehirn während der akuten Depression.

Zu den Figuren im einzelnen:
Figur a: CSF-Probe von Patient 36 - major depressive disorder, zweite Episode, weiblich, 58 Jahre
Figur b: CSF-Probe Patient 137 - major depressive disorder, zweite Episode, weiblich, 29 Jahre
Figur d: CSF-Proben Patient 27: Schizophrenie, paranoider Typus, chronisch, männlich, 39 Jahre; Patient 62: Schizophrenie, paranoider Typus, unspezifisch, weiblich, 37 Jahre; Patient 130: Schizophrenie, paranoider Typus, subchronisch, weiblich, 20 Jahre

### XI. Resümee

Borna Disease Virus (BDV) gilt als Prototyp einer neuen Familie von eingehüllten Negativstrang-RNA-Viren. BDV infiziert Nervenzellen, aber auch nicht-neuronale Zellen in Gehirn und Körper ohne Zellzerstörung. BDV-Infektionen persistieren. Sie kommen beim Menschen und vielen Haus- und Nutztieren vor. Humane BDV-Infektionen sind mit hoher Wahrscheinlichkeit an phasischen Gemütsstörungen beteiligt, beim Tier verursacht BDV phasische Verhaltensstörungen. Individuelle Risikofaktoren entscheiden über die Häufigkeit von Aktivierungen der latenten Infektion und damit über das Erkrankungsrisiko. Symptomfreie Träger gibt es in jeder Spezies. Die Diagnostik wird durch geringe Replikationsraten des Virus und lange Latenzphasen erschwert.
Die Erfindung ermöglicht die nahezu lückenlose Erfassung von Infektionen mit Borna Disease Virus (BDV) bei Mensch und Tier durch Kombination eines neu entdeckten Testparameters, CICs im Blutplasma, mit bereits bekannten Parametern, AG in weißen Blutzellen oder Blutplasma und AK im Plasma. Alle drei Parameter können mit einer einzigen Blutprobe (10 ml Citratblut) bestimmt werden.

Der in der Erfindung erstmalig beschriebene Nachweis von BDV-spezifischen CICs als einzigen längerfristig persistierenden Infektionsmarkern ermöglicht sowohl die Erfassung gesunder Träger (latenter Infektionen) als auch die Beurteilung von Infektionsverlauf und Therapieerfolg bei erkrankten Individuen.

## Patentansprüche

1. Verfahren zum Nachweis von Borna Disease Virus (BDV)-Infektionen, **dadurch gekennzeichnet, dass** an einer zu untersuchenden Körperflüssigkeitsprobe in der Körperflüssigkeit frei zirkulierende Immunkomplexe aus BDV-Antigenen und hieran angelagerten spezifischen Antikörpern, CIC genannt, durch einen geeigneten immunologischen Test nachgewiesen werden.

2. Verfahren nach Anspruch 1, **dadurch gekennzeichnet, dass** zur praktisch lückenlosen Erfassung persistenter BDV-Infektionen zusätzlich ein BDV-Antigennachweis, vorzugsweise auf das BDV-Nukleoprotein p40 (40kDa relatives Molekulargewicht, "N-Protein") und/oder auf das BDV-Phosphoprotein p24 (24kDa rel. Molekulargewicht, "P-Protein") und/oder ein Antikörpernachweis auf BDV-spezifische Antikörper durchgeführt wird.

3. Verfahren nach Anspruch 1 oder 2, **dadurch gekennzeichnet, dass** die Körperflüssigkeitsprobe eine Blut-, Urin- oder Liquorprobe ist und folgendermaßen behandelt wird:
(a) aus der Blutprobe wird eine Blutplasmafraktion isoliert und an der Blutplasma-, Liquor- oder Urinprobe werden unabhängig voneinander folgende Tests durchgeführt:
(1) ein Antigen-Nachweis auf BDV-Antigene,
(2) ein Antikörpernachweis auf BDV-spezifische Antikörper,
(3) ein CIC-Nachweis, vorzugsweise gemäß einem der Ansprüche 5 bis 9;
(b) aus der Blutprobe werden eine Blutplasmafraktion und eine Leukozytenfraktion präpariert und der Test gemäß (a)(1) wird im Falle einer Blutprobe an der Leukozytenfraktion und/oder der Blutplasmafraktion durchgeführt.

4. Verfahren nach Anspruch 3, **dadurch gekennzeichnet, dass** die zu untersuchende Körperflüssigkeitsprobe eine Blutprobe ist und dass alle Untersuchungen an einer Blutplasmaprobe durchgeführt werden.

5. Verfahren zum Nachweis in Körperflüssigkeiten zirkulierender BDV-spezifischer Immunkomplexe, CICs genannt, **dadurch gekennzeichnet, dass** folgende Schritte durchgeführt werden:
(1) Fixieren von monoklonalen oder polyklonalen BDV-spezifischen Antikörpern, die an die in den CICs enthaltenen Antigene spezifisch binden, über die Fc-Region in geeigneter Weise auf einem ggf. hierfür vorbereiteten Träger;
(2) Inkontaktbringen einer Körperflüssigkeits-, vorzugsweise Blutplasmaprobe, die auf Anwesenheit von CICs getestet werden soll, mit den fixierten Antikörpern;
(3) Inkontaktbringen eines Sekundärantikörpers einer anderen als der getesteten Spezies, vorzugsweise eines Ziegen-Anti-Spezies-Antikörpers, der spezifisch ist auf Antikörper der Spezies, deren Körperflüssigkeitsprobe verwendet wurde, mit der nach Schritten (1) und (2) behandelten Probe;
(4) Nachweis und/oder Bestimmung der Menge des Sekundärantikörpers durch ein geeignetes immunologisches Nachweisverfahren.

6. Verfahren nach Anspruch 5, **dadurch gekennzeichnet, dass** die in Schritte (1) verwendeten monoklonalen BDV-spezifischen Antikörper N- und/oder P-Protein-Antikörper sind.

7. Verfahren nach Anspruch 5 oder 6, **dadurch gekennzeichnet, dass** der Träger eine adsorptiv fixierende Kunststoff-Testplatte ist, dass diese Platte zunächst mit Sekundärantikörpern möglichst vollständig belegt wird, die gegen die Spezies, von der die CIC-Antigen-spezifischen Antikörper gewonnen wurden, spezifisch sind und dass daran anschließend die CIC-Antigen-spezifischen Antikörper auf die Sekundärantikörperschicht aufgebracht werden.

8. Verfahren nach Anspruch 5 oder 6, **dadurch gekennzeichnet, dass** die Fixierung der CIC-Antigen-spezifischen Antikörper in Schritt (1) des Verfahrens an polystyrolfixiertes C1q erfolgt.

9. Verfahren nach einem der Ansprüche 5 bis 8, **dadurch gekennzeichnet, dass** der Nachweis des Sekundärantikörpers gemäß Schritt (4) des Verfahrens über ein EIA- oder RIA-Verfahren erfolgt, vorzugsweise so, dass der Sekundärantikörper mit alkalischer Phosphatase gekoppelt wird und mit p-Nitrophenylphosphat mittels einer Farbreaktion visuell sichtbar oder mit optischen Detektoren auslesbar gemacht wird.

10. Diagnostischer Kit zum Nachweis von BDV-Infektionen, wobei der Kit BDV-spezifische monoklonale oder polyklonale Antikörper enthält, Mittel zum Inkontaktbringen dieser Antikörper mit einer Probe, von der vermutet wird, dass sie BDV-CICs enthält, sowie Mittel zum Nachweis der angelagerten Antigen-Antikörper-Komplexe, CICs genannt, über den Nachweis von Antikörpern der Spezies, deren Körperflüssigkeitsprobe verwendet wurde, insbesondere durch Sekundärantikörper einer anderen als der getesteten Spezies, vorzugsweise Ziegen-Anti-Spezies-Antikörper, die spezifisch sind auf Antikörper der Spezies, deren Körperflüssigkeitsprobe verwendet wurde.

11. Diagnostischer Kit nach Anspruch 10, wobei von der Probe zusätzlich vermutet wird, dass sie BDV-Antigene enthält und wobei der Kit zusätzlich Mittel zum Nachweis der angelagerten Antigene umfasst.

12. Diagnostischer Kit zum Nachweis von BDV-Infektionen nach Anspruch 10 oder 11, wobei der Kit mit BDV-Antigenen belegte BDV-spezifische monoklonale oder polyklonale Antikörper enthält, Mittel zum Inkontaktbringen dieser Antigen-belegten Antikörper mit einer Probe, von der vermutet wird, dass sie BDV-Antikörper enthält, und Mittel zum Nachweis der angelagerten Antikörper, insbesondere Sekundärantikörper, wie in Ansprüchen 5 und 10 genannt.

13. Diagnostischer Kit nach einem der Ansprüche 10 bis 12, **dadurch gekennzeichnet, dass** er eine Einheit umfasst, auf oder in der die BDV-spezifischen Antikörper immobilisiert vorliegen.

14. Diagnostischer Kit nach Anspruch 13, **dadurch gekennzeichnet, dass** die BDV-spezifischen Antikörper monoklonale oder polyklonale, von einer Spezies I gewonnene Antikörper sind, die **dadurch** immobilisiert sind, dass sie auf einen mit einem von einer anderen Spezies, nämlich Spezies II, gewonnenen Spezies II-anti-Spezies-I IgG beschichteten Träger, vorzugsweise in Form einer Festplatte oder eines Teströhrchens, aufgebracht und hierdurch fixiert sind.

15. Diagnostischer Kit nach Anspruch 14, **dadurch gekennzeichnet, dass** die Antikörper der Spezies I polyklonale oder monoklonale Mausantikörper, vorzugsweise P-Protein- und/oder N-Protein -spezifische monoklonale Mausantikörper, sind und dass die adsorptive Beschichtung des Trägers aus einem anti-Maus IgG, vorzugsweise einem Ziege-anti-Maus IgG, besteht.

16. Diagnostischer Kit nach Anspruch 13, **dadurch gekennzeichnet, dass** die BDV-spezifischen Antikörper über polystryrolgebundenes C1q fixiert bzw. immobilisiert sind.

17. Verwendung eines BDV-Antigengemisches, gewonnen aus Körperflüssigkeit und/oder Zellhomogenisat mit BDV erkrankter Tiere oder Menschen, das zumindest in seiner qualitativen Zusammensetzung einem Antigengemisch entspricht, welches mit Hilfe der unter der Nr DSM ACC 2334 hinterlegten Zellkultur gewonnen werden kann, zur Herstellung von Antikörpern für einen Kit gemäß einem der Ansprüche 10 bis 16 oder die Verwendung in einem Verfahren gemäß einem der Ansprüche 1 bis 9.

## Claims

1. Process for detecting Borna disease virus (BDV) infections, **characterized in that** in a body fluid specimen to be assayed, immune complexes composed of BDV antigens and specific antibodies attached thereto, known as CICs, and which circulate freely in the body fluid are detected by means of a suitable immunological assay.

2. Process according to Claim 1, **characterized in that** a BDV antigen detection, preferably for the BDV nucleoprotein p40 (relative molecular weight 40 kDa, "N protein") and/or for the BDV-phosphoprotein p24 (relative molecular weight 24 kDA, "P protein") and/or an antibody detection for BDV-specific antibodies is additionally carried out to identify persistent BDV infections virtually completely.

3. Process according to Claim 1 or 2, **characterized in that** the body fluid specimen is a blood, urine or spinal fluid specimen and is treated as follows:
(a) a blood plasma fraction is isolated from the blood specimen, and the following assays are carried out independently of one another on the blood plasma, spinal fluid or urine specimen:
(1) an antigen detection for BDV antigens,
(2) an antibody detection for BDV-specific antibodies,
(3) a CIC detection, preferably according to any of Claims 5 to 9;
(b) a blood plasma fraction and a leukocyte fraction are prepared from the blood specimen, and the assay in accordance with (a)(1) is carried out on the leukocyte fraction - in the case of a blood specimen - and/or on the blood plasma fraction.

4. Process according to Claim 3, **characterized in that** the body fluid specimen to be assayed is a blood specimen and all tests are carried out on a blood plasma specimen.

5. Process for detecting BDV-specific immune complexes, known as CICs, which circulate in body fluid, **characterized in that** the following steps are carried out:
(1) fixing, via the Fc region in a suitable manner on a carrier which may have been prepared for this purpose, monoclonal or polyclonal antibodies which specifically bind to the antigens contained in the CICs ;
(2) bringing into contact with the fixed antibodies a body fluid specimen, preferably a blood plasma specimen, which is to be assayed for the presence of CICs ;
(3) bringing into contact with the specimen treated in accordance with steps (1) and (2) a secondary antibody of a species other than the assayed species, preferably a goat-anti-species antibody, which is specific for antibodies of the species whose body fluid specimen was used;
(4) detection and/or measurement of the quantity of the secondary antibody by a suitable immunological detection process.

6. Process according to Claim 5, **characterized in that** the antibodies used in step (1) are monoclonal BDV-specific N and/or P protein antibodies.

7. Process according to Claim 5 or 6, **characterized in that** the support is an adsorptively fixing polymer assay plate, this plate first being occupied as completely as possible with secondary antibodies which are specific for the species from which the CIC-antigen-specific antibodies were obtained, and subsequently the CIC-antigen-specific antibodies are applied to the layer of secondary antibodies.

8. Process according to Claim 5 or 6, **characterized in that** the CIC-antigen-specific antibodies are fixed to polystyrene-fixed Clq in step (1) of the process.

9. Process according to any of Claims 5 to 8,
**characterized in that** the detection of the secondary antibody in accordance with step (4) of the process is done via an EIA or RIA process, preferably in such a way that the secondary antibody is coupled to alkaline phosphatase and is visualized with p-nitrophenyl phosphate by means of a color reaction or made selectable by means of optical detectors.

10. Diagnostic kit for detecting BDV infections, the kit comprising BDV-specific monoclonal or polyclonal antibodies, means for contacting these antibodies with a specimen suspected of containing BDV-CICs, and means for detecting the attached antigen-antibody complexes, known as (CICs) via the detection of antibodies of the species whose body fluid sample has been used, in particular by means of secondary antibodies of a species other than the assayed species, preferably goat-anti-species antibodies which are specific for antibodies of the species whose body fluid specimen has been used.

11. Diagnostic kit according to Claim 10, the sample additionally being suspected of containing BDV antigens, and the kit additionally comprising means for detecting the attached antigens.

12. Diagnostic kit for detecting BDV infections according to Claim 10 or 11, the kit comprising BDV-specific monoclonal or polyclonal antibodies occupied by BDV antigens, means for contacting these antigen-occupied antibodies with a specimen suspected of containing BDV antibodies, and means for detecting the attached antibodies, in particular secondary antibodies as mentioned in Claims 5 and 10.

13. Diagnostic kit according to any of Claims 10 to 12, **characterized in that** it comprises a unit on or in which the BDV-specific antibodies are present in immobilized form.

14. Diagnostic kit according to Claim 13, **characterized in that** the BDV-specific antibodies are monoclonal or polyclonal antibodies obtained from a species I which are immobilized by being applied, and thus fixed, to a support coated with a species II anti-species I IgG obtained from a different species, namely species II, preferably in the form of a solid plate or an assay tube.

15. Diagnostic kit according to Claim 14, **characterized in that** the antibodies from species I are polyclonal or monoclonal mouse antibodies, preferably P-protein- and/or N-protein-specific monoclonal mouse antibodies, and **in that** the adsorptive coating of the support is composed of an anti-mouse IgG, preferably a goat anti-mouse IgG.

16. Diagnostic kit according to Claim 15, **characterized in that** the BDV-specific antibodies are fixed, or immobilized, via polystyrene-bound Clq.

17. Use of a BDV antigen mixture, obtained from body fluid and/or cell homogenate of BDV-diseased animals or humans, which homogenate corresponds at least with regard to its qualitative composition to an antigen mixture obtainable with the aid of the cell culture deposited under the No. DSM ACC 2334, for raising antibodies for a kit according to any of Claims 10 to 16 or the use in a process according to any of Claims 1 to 9.

## Revendications

1. Procédé pour détecter les infections causées par le virus de la maladie de Borna (BDV), **caractérisé en ce qu'**on détecte par un test immunologique approprié sur un échantillon de liquide corporel, les immuno-complexes d'antigènes BDV et les anticorps spécifiques appelés CIC associés à ceux-ci circulant librement dans le liquide corporel.

2. Procédé selon la revendication 1, **caractérisé en ce que** pour enregistrer pratiquement sans lacunes des infections BDV persistantes on effectue en outre une détection d'antigènes BDV de préférence sur la nucléoprotéine BDV p40 (masse moléculaire relative de 40kDa, «Protéine N ») et/ou sur la phosphoprotéine BDV p24 (masse moléculaire relative de 24kDA, « Protéine P ») et/ou une détection d'anticorps sur des anticorps BDV spécifiques.

3. Procédé selon la revendication 1 ou 2, **caractérisé en ce que** l'échantillon de liquide corporel est un échantillon de sang, d'urine ou de liqueur et est traité selon les mesures suivantes :
(a) on isole de l'échantillon de sang une faction de plasma sanguin et sur l'échantillon de plasma sanguin, de liqueur ou d'urine on effectue les tests suivants indépendants les uns des autres :
(1) une détection d'antigène sur l'antigène BDV,
(2) une détection d'anticorps sur l'anticorps BDV spécifique,
(3) une détection de CIC, de préférence selon l'une des revendications 5 à 9 ;
(b) à partir de l'échantillon de sang, on prépare une fraction de plasma sanguin et une fraction de leucocytes et le test selon (a) (1) est dans le cas d'un échantillon de sang effectué sur la fraction de leucocytes et/ou la fraction de plasma sanguin.

4. Procédé selon la revendication 3, **caractérisé en ce que** l'échantillon de liquide corporel à examiner est un échantillon de sang et **en ce que** tous les examens sont effectués sur un échantillon de plasma sanguin.

5. Procédé pour détecter des immuno-complexes BDV spécifiques appelés CICs, **caractérisé en ce qu'**on effectue les étapes suivantes :
(1) fixation d'anticorps BDV spécifiques monoclonaux ou polyclonaux qui se lient à l'antigène CICs spécifique obtenu au dessus de la région Fc de façon appropriée sur éventuellement un support préparé à cet effet ;
(2) mise en contact d'un liquide corporel, de préférence un échantillon de plasma sanguin qui doit être testé pour la présence de CICs, avec les anticorps fixés ;
(3) mise en contact d'un anticorps secondaire autre que celui de l'espèce testée, de préférence un anticorps anti-espèce de chèvre, qui est spécifique de l'anticorps de l'espèce, dont l'échantillon de liquide corporel a été utilisé, avec l'échantillon traité selon les étapes (1) et (2) ;
(4) détection et/ou détermination de la quantité de l'anticorps secondaire selon un procédé de détection immunologique approprié.

6. Procédé selon la revendication 5, **caractérisé en ce que** les anticorps monoclonaux BDV spécifiques utilisés dans l'étape (1) sont des anticorps protéine N et/ou des anticorps protéine P.

7. Procédé selon la revendication 5 ou 6, **caractérisé en ce que** le support est une plaque de test en matière synthétique à fixation adsorbante, **en ce que** cette plaque est d'abord recouverte de façon la plus complète possible par des anticorps secondaires qui sont spécifiques contre les espèces, à partir desquelles les anticorps spécifiques antigène CIC ont été obtenus et ensuite les anticorps spécifiques antigène CIC sont appliqués sur la couche d'anticorps secondaire.

8. Procédé selon la revendication 5 ou 6, **caractérisé en ce que** la fixation des l'anticorps spécifique antigène CIC dans l'étape (1) du procédé a lieu sur du C1q fixé sur du polystyrène.

9. Procédé selon l'une des revendications 5 à 8, **caractérisé en ce que** la détection de l'anticorps secondaire selon l'étape (4) du procédé est effectuée selon un procédé EIA ou RIA, de préférence de telle sorte que l'anticorps secondaire soit couplé avec de la phosphatase alcaline et avec du phosphate de p-nitrophényle et soit visible au moyen d'une réaction colorante ou lisible au moyen de détecteurs optiques.

10. Kit de diagnostic pour détecter les infections de BDV, dans lequel le kit renferme des anticorps spécifiques BDV monoclonaux ou polyclonaux, un moyen pour mettre en contact ces anticorps avec un échantillon qui est présumé contenir du BDV-CICs, ainsi qu'un moyen pour détecter le complexe associé antigène-anticorps appelé CICs, par la détection d'anticorps des espèces de l'échantillon de liquide corporel pour lesquels il a été utilisé, en particulier au moyen d'anticorps secondaires d'une autre espèce que celle testée, de préférence des anti-corps anti-espèce de chèvre, qui sont des anticorps spécifiques de l'espèce, de l'échantillon de liquide corporel qui a été utilisé.

11. Kit de diagnostic selon la revendication 10, dans lequel l'échantillon est également présumé contenir l'antigène BDV et dans lequel le kit comprend également un moyen pour détecter l'antigène associé.

12. Kit de diagnostic pour détecter les infections BDV selon la revendication 10 ou 11, dans lequel le kit contient les anticorps spécifiques de BDV monoclonaux ou polyclonaux recouverts d'antigènes BDV, des moyens de mise en contact des anticorps recouverts d'antigènes avec un échantillon présumé contenir un anticorps BDV et des moyens pour détecter l'anticorps associé, en particulier l'anticorps secondaire tel que mentionné dans les revendications 5 et 10.

13. Kit de diagnostic selon l'une des revendications 10 à 12, **caractérisé en ce qu'**il comprend une unité sur laquelle ou dans laquelle des anticorps spécifiques de BDV se trouvent immobilisés.

14. Kit de diagnostic selon la revendication 13, **caractérisé en ce que** les anticorps spécifiques de BDV monoclonaux ou polyclonaux, sont des anticorps obtenus à partir d'une espèce I qui sont immobilisés de façon à être appliqués et fixés sur un support de préférence sous forme de plaquette rigide ou un tube de test en étant recouvert par une espèce II-anti-espèce I Ig G obtenu à partir d'une autre espèce, à savoir une espèce II.

15. Kit de diagnostic selon la revendication 14, **caractérisé en ce que** les anticorps de l'espèce 1 sont des anticorps de souris polyclonaux ou monoclonaux de préférence des anticorps de souris spécifiques P-protéine et/ou N-protéine monoclonaux et la couche adsorbante du support est constituée d'un Ig G anti-souris, de préférence d'un Ig G chèvre-anti-souris.

16. Kit de diagnostic selon la revendication 13, **caractérisé en ce que** les anti-corps spécifiques de BDV sont fixés ou immobilisés sur du C1q lié par du polystyrène.

17. Utilisation d'un mélange d'antigènes de BDV, obtenu à partir de liquide corporel et/ou d'un homogénéisat de cellules avec du BDV d'animaux ou d'humains malades qui comprend au moins un mélange d'antigènes dans sa composition qualitative, qui peut être obtenu au moyen de la culture de cellules déposée sous le n°DSM ACC 2334, pour la préparation d'anticorps pour un kit selon l'une des revendications 10 à 16 ou l'utilisation dans un procédé selon l'une des revendications 1 à 9.
